# EUROPEAN PATENT APPLICATION

(11) **EP 3 875 433 A1**
(43) Date of publication of application: **08.09.2021**
(21) Application number: 19877759.1
(22) Date of filing: 04.11.2019
(51) Int. Cl.: C01G 49/08, B82Y 30/00, B82Y 40/00, A61K 47/02, A61K 38/16, A61K 31/704, A61P 35/00, A61K 49/18, A61B 5/055

(54) **MAMC-MEDIATED BIOMIMETIC NANOPARTICLES**

(30) Priority: 02.11.2018 ES 201831064
(71) Applicant: Universidad de Granada, 18071 Granada (ES)
(72) Inventor: JIMÉNEZ LÓPEZ, Concepción, 18071 Granada (ES); VALVERDE TERCEDOR, Carmen, 18071 Granada (ES); PEIGNEUX NAVARRO, Ana, 18071 Granada (ES); JABALERA RUZ, Ylenia María, 18071 Granada (ES); PRAT, María, 28100 Vercelli (IT); OLTOLINA, Francesca, 28100 Vercelli (IT)
(74) Representative: Hoffmann Eitle
(86) International application number: PCT/ES2019/070747
(87) International publication number: WO 2020/089505

(57) **Abstract**

Biomimetic magnetic nanoparticles comprising MamC.

The present invention provides superparamagnetic biomimetic nanoparticles comprising magnetite, which can be produced using a scalable process. In addition, these nanoparticles exhibit promising properties, since, if functionalized, they can be converted into drug carriers or contrast agents for obtaining clinical images. They can also be used in clinical setting to purge bone marrow, as molecule separators, and/or for environmental applications as biosensors. These nanoparticles, coupled to a drug, can be encapsulated in liposomes, thereby obtaining magnetoliposomes, which can be functionalized for use in the targeted delivery/release of drugs. In addition, mixtures of magnetoliposomes (functionalized or not with a targeting agent) and functionalized biomimetic magnetic nanoparticles or liposomes containing mixtures of functionalized BMNPs and MNPs can be used to combine different treatments, such as, for example, targeted delivery/release of drugs and hyperthermia.

## Description

### Technical Field

The present invention relates to compositions comprising biomimetic magnetic nanoparticles. The magnetic nanoparticles can be used as a medicament, in particular, as a medicament for the treatment of diseases with an associated marker which can be targeted, such as, for example, cancer.

### Background Art

Nanotechnology, and in particular magnetic nanoparticle (MNPs) production, generates millions of dollars every year in the USA. Nanotechnology application is based upon the fact that they can be easily manipulated by applying an external magnetic field and thus, can be directed toward the target site by an external controller (Arakaki *et al.,* 2014; Prozorov *et al.,* 2013). To date, these particles are used in a wide range of applications from ferrofluids and magnetic storage to the clinical/scientific area, such as detection of nucleotide polymorphism (Maruyama *et al.,* 2004, 2007; Matsunaga *et al.,* 2007), cell separation (Matsunaga *et al.,* 2007), DNA isolation and purification (Ota *et al.,* 2006), contrast agent in magnetic resonance imaging (MRI; Lisy *et al.,* 2007), early diagnosis, drug carrier for targeted chemotherapy (Sun *et al.,* 2008) and hyperthermia cancer treatments, understood as the thermal damage induced by the rotation of localized nanoparticles (Alphandéry *et al.,* 2011).

For these applications, especially in clinical setting, one of the most important requirements is for the MNPs used as nanocarriers to respond, as efficiently as possible, to the external magnetic field applied to guide such nanocarrier to the target site (Prozorov *et al.,* 2013). Such a response depends on the magnetic moment per particle, which for superparamagnetic, crystalline stoichiometric magnetite magnetic nanoparticles, in fact depends on the size of the MNP. For magnetite, the ideal nanoparticle size required to comprise only a single magnetic domain is between 30 to 120 nm. Most of the superparamagnetic nanoparticles already commercialized are small (< 30 nm) and thus, their magnetic moment could be increased if larger MNPs were used. Above 120 nm, magnetic nanoparticles comprise multiple domains and their magnetic moment is unstable and depends upon the orientation of these nanoparticles. Size is also important when hyperthermia treatments are in play. In fact, the heating power generated per particle unit mass upon application of an alternating external magnetic field is directly related to the amount of iron in the MNP, and it should be as high as possible to keep the applied magnetic field within the ranges accepted in clinical setting and a low dose of MNPs. Moreover, the magnetic nanoparticles intended to be used in clinical setting must also be biocompatible and not pose any risk associated with the doses that has to be applied. Another important requirement for the MNPs is that they provide at their surface functional groups that allow functionalization/release of drugs based on external stimuli, such as changes in the environmental pH. For this purpose, most of the commercial MNPs are coated with compounds such as polyethylene glycol and organic acids. This procedure, not only introduces additional steps in the MNP production process (obviously increasing the preparation time and overall cost), but also, the coating may shield the magnetic core and thus interfere with the magnetic response of the nanoparticle to an applied external magnetic field (response which is already suboptimal due to the small size thereof). Therefore, most commercial MNPs have drawbacks that need to be overcome in order to be able to use MNPs as efficient nanocarriers.

Magnetite (Fe²⁺ and Fe³⁺ oxide, Fe₃O₄) is a mineral found in various environments, from igneous to metamorphic rocks to many sedimentary environments, both terrestrial and extraterrestrial (Thomas-Keprta *et al.,* 2000). It has also been found in high phyla organisms like those with a migratory behavior and in chitons. Inorganically, magnetite can form as a primary phase from solutions containing Fe²⁺ and/or Fe³⁺ (coprecipitation method and oxide-reduction method) the pH of which is raised through the addition of chemical compounds (i.e.: Arató *et al.,* 2005; Perez-Gonzalez *et al.,* 2010; Prozorov *et al.,* 2007; Schwertmann and Cornell, 2000). This is the most widely used method to produce magnetic nanoparticles. It is relatively easy to perform, the nanoparticles are produced at room temperature and large amounts of material can be obtained in each batch. The main disadvantage is that these nanoparticles are usually of a small size (<30 nm) and thus, they have a small magnetic moment per particle, which increases the doses to be used and may pose a risk.

Magnetites can also form through the transformation of precursors, usually at high temperatures (Jimenez-Lopez *et al.,* 2012). An advantage of this approach is that cubic and well crystallized magnetites can be formed. However, a significant disadvantage of this approach is that this protocol is very expensive and the size of the nanoparticles is hard to control. Magnetite nanoparticles obtained by this process are usually either fairly small (<30 nm) or comprise multiple domains (>120 nm).

Also, magnetite can form biologically, either by a bio-induced mineralization (BIM) process or a bio-controlled mineralization (BCM) process (Bazylinski and Frankel, 2004). Magnetite formation through BIM is the result of an organism's metabolic activity and the subsequent chemical reactions mediated by the metabolic products. Minerals formed through BIM are indistinguishable from minerals formed inorganically under these conditions (Perez-Gonzalez *et al.,* 2000). These BIM nanoparticles are not frequently used for nanotechnology applications.

On the contrary, magnetite nanoparticles formed by magnetotactic bacteria are the result of a biomineralization process exquisitely controlled at the genetic level (BCM), which makes these particles the ideal magnetic nanoparticles. They are ideal because they present very specific characteristics such as perfect crystalline structures, high chemical purity, non-equilibrium morphology, and a narrow size distribution (Bazylinski and Frankel, 2004), which causes these crystals to comprise a single magnetic domain and to have foreseeable and stable magnetic properties (Amemiya *et al.,* 2007; Prozorov *et al.,* 2013). Also, another advantage is that they are biocompatible. Therefore, these particles are in high demand, especially in clinical setting.

However, these nanoparticles cannot be commercialized because magnetotactic bacterial culture cannot be scaled to industrial levels due to the very slow growth and the very stringent nutritional requirements. To solve this problem, different alternatives are being explored in order to improve magnetosome-like nanoparticle production yields. Basically, three strategies are being followed. The first one is to try to transform less fastidious, non magnetotactic microorganisms into magnetotactic bacteria. In this sense, the work of Kolinko *et al.* (2014) is particularly relevant. These authors demonstrated, for the very first time, how the insertion of specific genes of *Magnetospirillum gryphiswaldense* in *Rhodospirillum rubrum* caused the latter to produce heterologous magnetosomes. They also showed that when genes involved in the production of these heterologous magnetosomes in *Rhodospirillum* were silenced, the alterations that occurred were comparable to those that occurred in *M. gryphiswaldense.* With their fine work, these authors opened the door to the possibility of scaling up the production of heterologous magnetosomes in microorganisms that are easier to culture. However, although very promising, the stability of the mutant and the real scaling up of these heterologous magnetosomes have yet to be proven and these aspects were not explored by these authors.

The second alternative is to use recombinant DNA technology to silence or overexpress the genes of interest in specific strains. In this sense, patent US2010/0292495 A1 which intends to obtain recombinant magnetite nanoparticles by controlling the expression of *mamG, mamF, mamD,* and *mamC* genes in magnetotactic bacteria, can be mentioned.

Finally, the third alternative involves biomimetic approaches, i.e., learning from nature, which can inspire new strategies to produce advanced functional materials. Therefore, in order to chemically produce magnetosome-like crystals whose production can be scaled up to industrial levels, several magnetosome proteins, both full length proteins expressed as recombinant proteins and synthetic peptides, have been tested in different *in vitro* magnetite production experiments. Magnetite nanoparticles with different magnetic properties compared to those from inorganic chemical precipitation have been obtained as a result of the mediation of these proteins. In this sense, most of the work performed up until now involved using full length Mms6 protein from different *Magnetospirillum* species to obtain magnetite nanoparticles *in vitro* (Arakaki *et al.,* 2010, 2014; Amemiya *et al,.* 2007; Prozorov *et al.,* 2007; Galloway *et al.,* 2012; Bird *et al.,* 2016). Most of the magnetic nanoparticles obtained through the mediation of these proteins are superparamagnetic nanoparticles of about 20 nm in size. Therefore, they display little advantages with respect to the commercial nanoparticles already on the market. By means of the biotemplating technique, bigger nanoparticles are obtained with Mms6 (Galloway *et al.,* 2012b; Bird *et al.,* 2016), but the magnetic properties of these nanoparticles have not been studied and the process of scaling up same, which is probably very difficult and expensive, has not been addressed either. The MmsF protein from *Magnetospirillum magneticum* AMB-1 is another potential candidate for mediating the *in vitro* formation of biomimetic magnetite nanoparticles of 80-90 nm in size (Rawlings *et al.,* 2014). These nanoparticles could comprise only a single magnetic domain, but the magnetic properties of these nanoparticles have not been studied and well-characterized enough so as to determine whether or not they could be of use in nanotechnology. All these experiments, nevertheless, have been performed using only one recombinant protein in the aqueous solution in which magnetite forms.

Biomimetic nanoparticles have also been produced by using chimeras constructed with synthetic peptides of magnetosome proteins attached to fusion proteins (Nudelman *et al.,* 2016 and 2018). Some of these peptides are already patented (WO 2017153996). When MamC-loop peptides are used, nanoparticles larger in size compared to those produced in the presence of other peptides or in the absence of any peptides are obtained. However, size distribution of the nanoparticles, and thus, nanoparticle heterogeneity, was larger than that exhibited by the nanoparticles produced in the presence of the full length MamC protein expressed as a recombinant.

The research team of Prof. Jimenez-Lopez has made significant progress in the production of biomimetic nanoparticles. In fact, it is the only group working with MamC from *Magnetococcus marinus* MC-1, the most abundant magnetosome protein in most magnetotactic bacteria. Also, it is the only research group that has expressed and purified as recombinant proteins the three proteins that control the size and/or morphology of magnetosomes in MC-1 (MamC, Mms6 and Mms7). MamC from *Magnetococcus marinus* MC-1 has emerged as a strong candidate to produce novel biomimetic magnetic nanoparticles (BMNPs). They display a larger size (-40 nm) compared to most commercial MNPs (≤ 30 nm) and exhibit (1) a higher blocking temperature while being superparamagnetic at room temperature, (2) a slower increase of magnetization with temperature, and (3) a Verwey transition temperature. All these characteristics are consistent with nanoparticles with a larger structure and a larger magnetic moment per particle, compared to other inorganic nanoparticles and/or most Mms6-mediated nanoparticles. These characteristics cause them to behave as if they were non magnetic at room temperature in the absence of an external magnetic field, which prevents agglomeration, while efficiently responding to an external magnetic field, thus increasing the efficiency of the magnetic guidance. Moreover, previous results from the group showed that MamC confers new surface properties to these BMNPs, in particular, the isoelectric point (iep) at pH 4.4. This is important because the BMNPs are negatively charged at physiological pH and can be functionalized with molecules, such as doxorubicin (DOXO), that are positively charged at that pH, by electrostatic interactions. Moreover, as the pH becomes acidic (as it naturally happens in tumor microenvironments), such electrostatic interaction weakens and the molecule is released from the BMNPs. These BMNPs are cytocompatible and biocompatible. The properties of the resultant nanoparticles depend on the type of protein introduced in the solution prior the formation of said nanoparticle and/or the relative concentration of the different protein(s) used.

However, the process for forming the biomimetic magnetite nanoparticles using MamC leads to the formation of siderite [Valverde-Tercedor *et al.,* 2015 (magnetite >90% + siderite <10%)] or goethite [Lopez-Moreno *et al.,* 2017 (magnetite 85% + goethite 15%)] as an unwanted product. These mineral phases (siderite and/or goethite), once produced and due to their nanoscale size, are difficult, if not impossible, to eliminate from the mixture. Thus, there is a need for a method which produces only magnetite and not other phases. Further, there is a need to develop a composition comprising a substantially pure mineral phase of magnetite nanoparticles (≥ 95%), wherein the resultant nanoparticles are superparamagnetic, comprise a single magnetic domain, and have surface properties that allow functionalization with different molecules without the need of further treatment after production processes.

Nanoparticles can be envisioned as efficient nanocarriers for medicaments, especially when functionalized with molecules (such as antibodies, aptamers, ligands for cell surface receptors) capable of recognizing specific markers associated to a disease. This allows achieving large local amounts of medicament and low systemic exposure, thus reducing treatment toxicity by increasing its efficacy (Brigger *et al.,* 2001; De Jong and Borm, 2008; Singh and Lillard, 2009).

### Description of the Figures

**Figure 1****:** CLUSTAL O (1.2.1) multiple sequence alignment of Mms6 in different magnetotactic bacteria.
**Figure 2**: SDS-PAGE gel of purified MamC (lane 3) and Mms6 (lane 5). *E. coli* TOP10 lysates before the purification of MamC (lane 2) and Mms6 (lane 4). Lane 1, molecular weight marker (KDa).
**Figure 3**: Magnetite crystals synthesized in the presence of MamC (10 µg/mL) (BMNPs): (A) TEM images, (B) crystal size distribution. Insert: Modeling of BMNPs from HRTEM data by using SHAPE v7.3 Magnetite crystals synthesized in the absence of any protein (inorganic magnetite: MNPs): (C) TEM images, (D) crystal size distribution.
**Figure 4****:** HRTEM images of inorganic magnetite nanoparticles (A and B) and magnetite nanoparticles produced in the presence of MamC (C, D, and E). Dotted lines represent the crystal faces and solid lines represent the crystallographic directions.
**Figure 5****:** HRTEM images of magnetite nanoparticles produced in the presence of Mms6 (A, B) and magnetite nanoparticles produced in the presence of MamC and Mms6 (C and D).
Dotted lines represent the crystal faces and solid lines represent the crystallographic directions.
**Figure 6****:** (A) ζ-potential of MNPs and BMNPs, (B) Thermogravimetric analyses of MNPs and BMNPs, (C) Hysteresis cycle of BMNPs and MNPs at 300 K, (D) ZFC-Wand FC-C of MNPs and BMNPs. Blocking temperature (T_{B}) and irreversibility temperature (Tᵢᵣᵣ) are indicated in the figure for each sample.

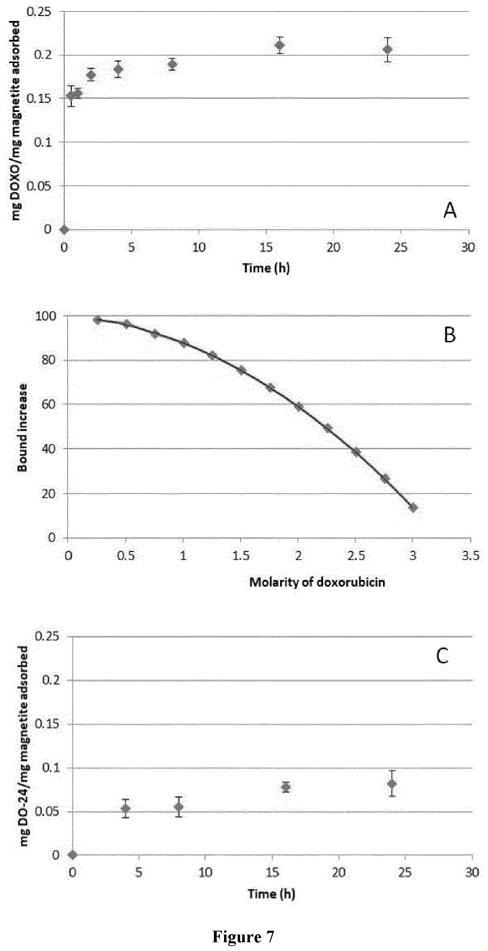

**Figure 7****:** Adsorption isotherm of Doxorubicin (DOXO) (A: kinetics; B: dose-dependency of adsorption. Saturation is achieved at 1 mmol DOXO per gram of nanoparticles) and DO-24 monoclonal antibody (C) on magnetite nanoparticles.
**Figure 8**: DOXO desorption profile.

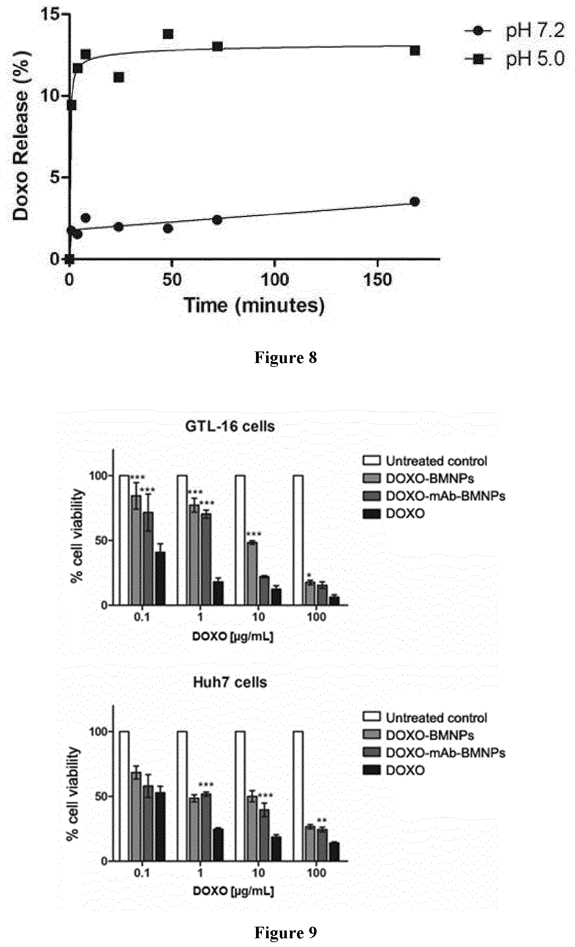

**Figure 9****:** Effect of the non-functionalized biomimetic nanoparticles and of ternary nanoparticles (functionalized with Doxorubicin [DOXO] and with DO-24 monoclonal antibody) on the viability of human tumor cells expressing the Met receptor, Met/HGF-R+ GTL-16, and not expressing the Met receptor, Met/HGF-R- Huh7. Non-functionalized nanoparticles reduced cell viability only to 0-95%. Doxo [µg/ml] indicates the DOXO concentration in each sample and the data expresses cell viability compared to the (untreated) control at the same time interval. At a DOXO concentration of 10 µg/ml, the ternary nanoparticles were significantly more toxic for GTL-16 than for Huh7, compared to the non-functionalized nanoparticles. Cell viability was measured in a MTT assay after 3 days of treatment.

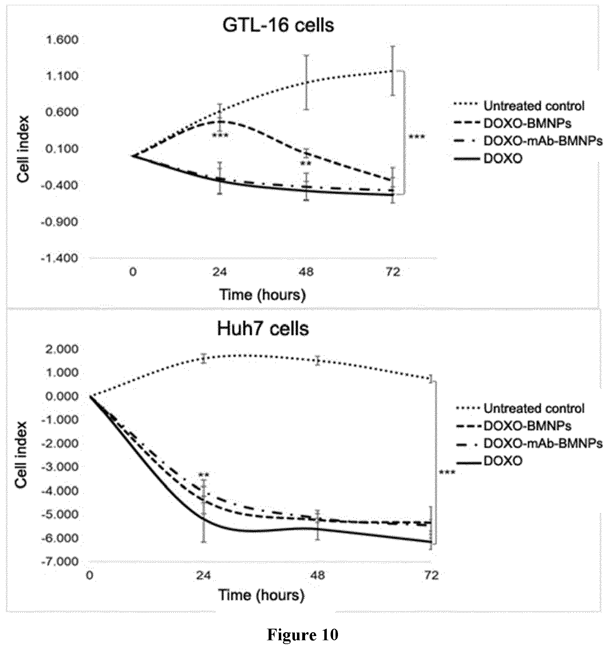

**Figure 10****:** Real time toxicity of the non-functionalized biomimetic nanoparticles, binary nanoparticles MNPs (DOXO-MNPs, -----) and ternary nanoparticles (-·-) on the Met/HGF-R+ GTL-16 cells and Met/HGF-R- Huh7 cells. The presence of DO-24 mAbs significantly increases the toxicity of the ternary nanoparticles compared to that of the binary nanoparticles in GTL-16 cells, while no differences were observed in those cells that did not express the Met receptor (Huh7). Cell index, indicative of cell viability, was measured in a XCELLIGENCE assay.

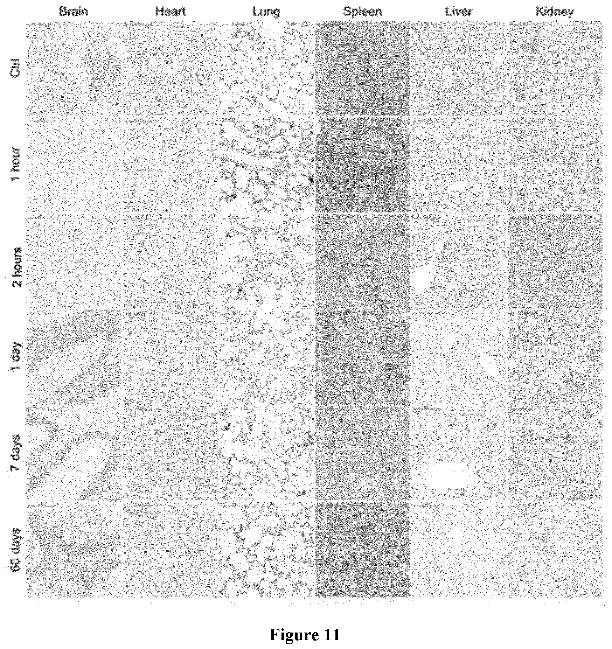

**Figure 11****:** Histological analysis of different organs from BALB/c female mice injected intravenously with biomimentic nanoparticles (10 µg/g of mouse). The mice were sacrificed at different times after injection (1h, 4h, 1d, 7d and 60d) and organs processed by means of Prussian Blue Iron staining and Hematoxylin-Eosin staining. Small amounts of iron, which decreased after 60 days, were detected in lungs. In spleens which already contain iron normally, the levels of iron increased one hour after injection and it then decreased in the following days, returning to normal levels after 60 days. In other organs (brain, heart, liver, and kidney), a slight increase in iron was detected in the first day and it decreased subsequently. The biomimetic nanoparticles are highly biocompatible *in vivo.*

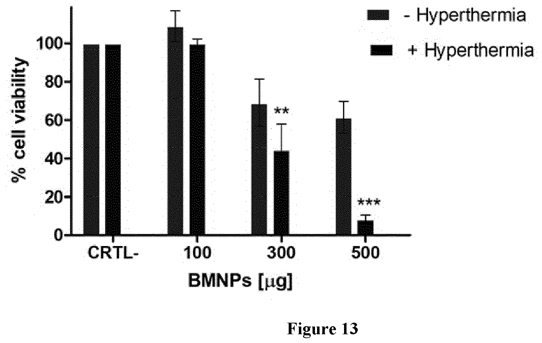

**Figure 13****: (a, b)** Cytocompatibility analyzed by MTT assay of the BMNPs (0.1, 1, 10 and 10 µg/mL) on 4T1 cells in the absence/presence of a gradient magnetic field after 72 h. (c) Analysis of potential ROS production in the presence of different concentrations (0.1, 1, 10 and 10 µg/mL) of the BMNPs on 4T1 cells, in the absence/presence of a gradient magnetic field, by confocal microscopy. The release of ROS (green). Menadione (100 µM) was used as a positive control. Fixed and permeabilized cells were stained for cytoskeletal actin with TRITC-phalloidin (red) and nuclei with TO-PRO3 (blue).

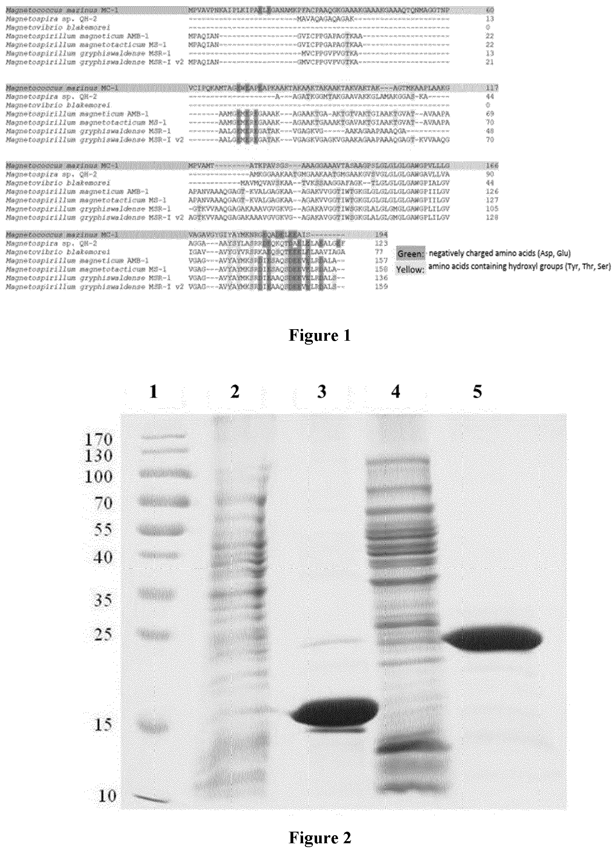

**Figure 2****:** Cytocompatibility/cytotoxicity of BMNPs (100, 300, 500 µg) on 4T1 cells in the absence/presence of an alternating magnetic field after 20 min.

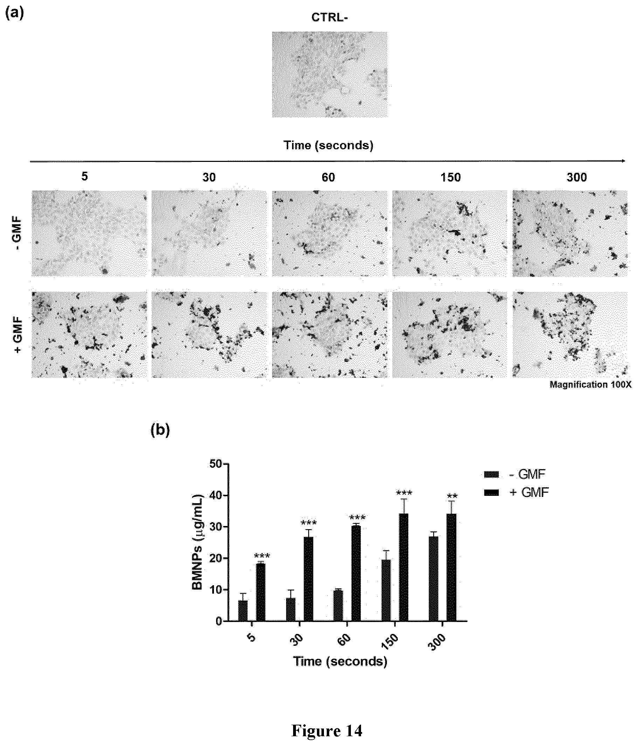

**Figure 14****. (a)** Qualitative (Prussian blue) and (b) quantitative (potassium thiocyanide) analyses of the interaction of BMNPs (100 µg/mL) with 4T1 cells at different times (5 and 30 seconds, 1, 2.5 and 5 minutes) in the absence (- GMF) and presence (+ GMF) of a gradient magnetic field. Untreated cells were used as negative control (CTRL-).

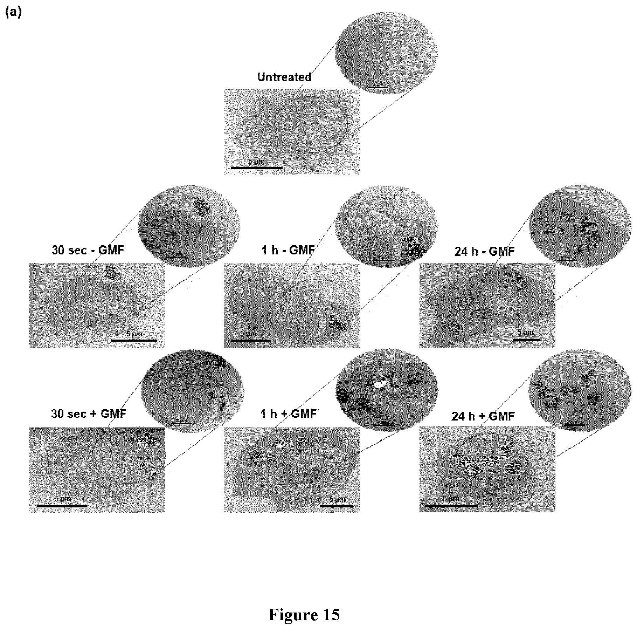

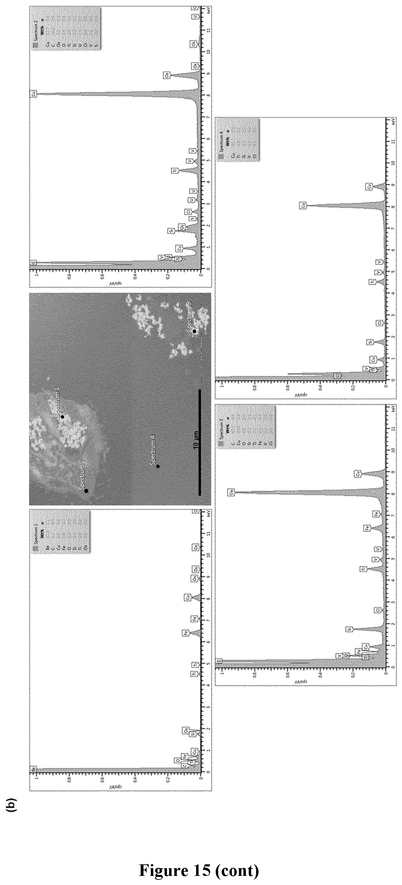

**Figure 15****: (a)** TEM micrographs and **(b)** microanalysis by energy dispersive X-ray (EDX) spectroscopy of 4T1 cells incubated with the 100 µg/mL of BMNPs in the absence/presence of a gradient magnetic field for 30 seconds, 1 and 24 h. The micrographs are representative of alternate serial cuts of the cell pellets of each sample.

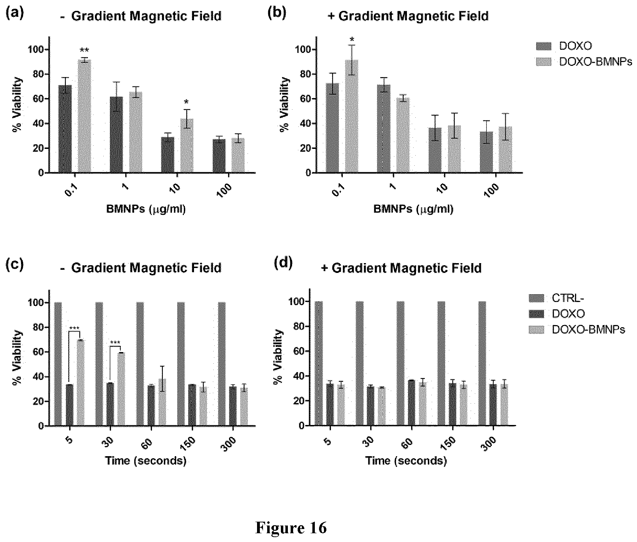

**Figure 16****:** Cytotoxic activity analyzed by MTT assay of DOXO-BMNPs and soluble DOXO (as a positive control) on 4T1 cells in the absence/presence of a gradient magnetic field. The cytotoxicity was analyzed **(a, b)** as a function of DOXO concentration (0.1, 1, 10, and 100 µg/mL), and **(c, d)** as a function of time (5, 30, 60, 150 and 300 seconds). In all the experiments untreated cells (CTRL-), receiving medium without nanoparticles, were taken as reference value (100%) of viable cells to which the values of treated cells refer. The data is the average of 3 experiments performed in triplicates.

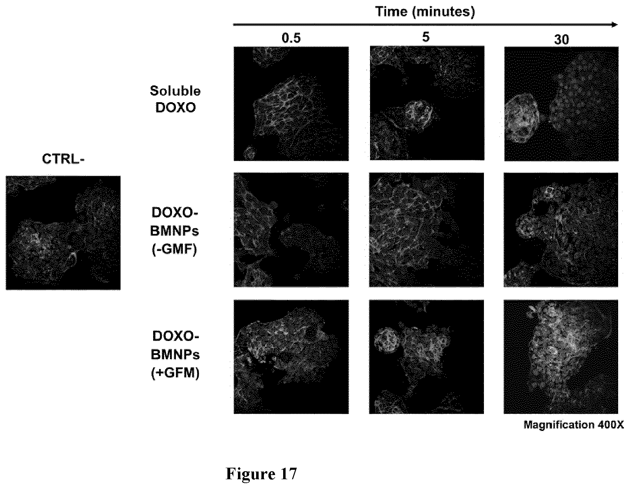

**Figure 17****:** Analysis of the interaction of DOXO-BMNPs with 4T1 cells at different times (0.5, 5 and 30 seconds, 1, 2.5 and 5 minutes) in the absence (- GMF) and presence (+ GMF) of a gradient magnetic field by confocal microscopy. Soluble DOXO was used as a positive control. Fixed and permeabilized cells were stained for cytoskeletal actin with FITC-phalloidin (green), nuclei with TO-PRO3 (blue) and DOXO itself emits fluorescence (red).

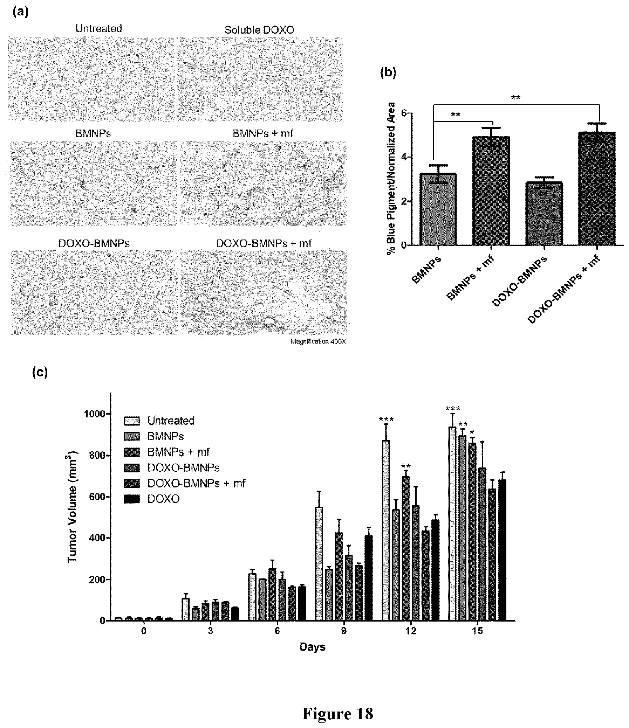

**Figure 18****: (a)** Antitumor effect of DOXO-BMNPs or non-functionalized BMNPs +/- gradient magnetic field application on the growth of 4T1 tumors in female BALB/c mice (n = X). Each treatment was administered intravenously 5 times, every 3-4 days, at a dose of 2 mg DOXO/kg mouse body weight or comparable amounts of BMNPs (300 µg BMNPs/g body weight). The total dose of DOXO in the treated groups was 10 mg/kg. **(b)** Tumor biodistribution profiles (Prussian blue staining) and **(b)** particles quantification in the tumor site performed for the different treatments at the end (day 15) of the experiment.

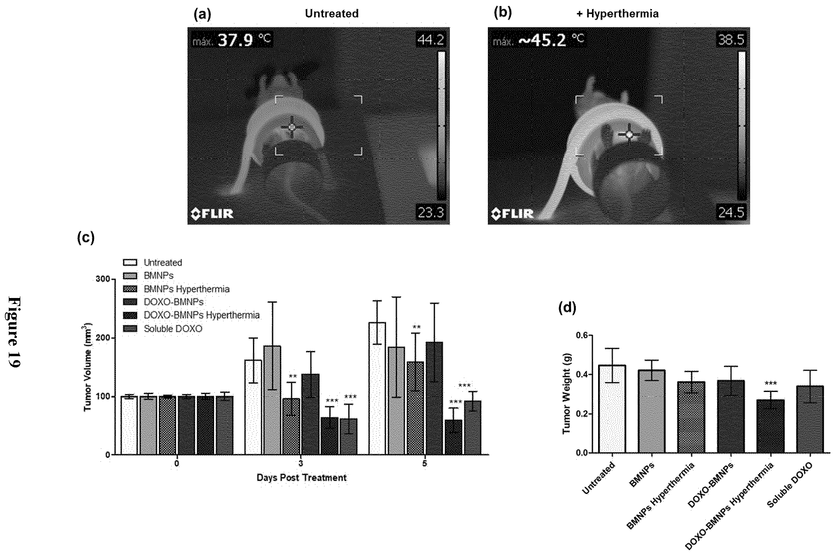

**Figure 19****:** *In vivo* hyperthermia under an alternating magnetic field. Images of groups (a) untreated and (b) treated with the alternating magnetic field taken with a thermal camera. **(b, d)** Antitumor effect of DOXO-BMNPs or non-functionalized BMNPs +/- alternating magnetic field (hyperthermia) application on the growth of 4T1 tumors in female BALB/c mice (n = 6). Each group received one intratumor injection of 3 mg BMNPs/mouse the first day of treatment. For the groups injected with soluble DOXO or DOXO-BMNPs, the dose of DOXO (either soluble or adsorbed on the BMNPs) was 80 µg/mouse.

### Brief Description of the Invention

The present invention provides a method for producing a composition comprising a substantially pure mineral phase of superparamagnetic biomimetic magnetite (BMNPs, ≥95% of total solid), said method comprising the following steps: (**a**) preparing a carbonate solution, (**b**) adding FeCl₃ to the carbonate solution, (**c**) adding MamC and, optionally, Mms6 to the solution obtained in step (b), (**d**) incubating the solution obtained in step (c) for at least 30 minutes, (**e**) adding Fe(ClO₄)₂ to the solution obtained in step (d), and (**f**) adjusting the pH of the solution obtained in step (e) to 9 using a base; wherein, the method is performed at 25°C and 1 atmosphere of pressure and all the solutions are previously deoxygenated. In the abovementioned method, the concentrations of the stock proteins must be in the following range: [MamC] 2-5 mg/mL, [Mms6] and [Mms7] > 1 mg/mL.

Further, the present invention provides a composition comprising: (i) a substantially pure mineral phase of superparamagnetic magnetite,(ii) MamC, and(iii) optionally, Mms6; wherein, at least components (i) and (ii) form superparamagnetic magnetic nanoparticles containing up to 5 wt% of MamC, with a mean particle size between 30 and 120 nm.

Further, the present invention provides a formulation for preparing magnetoliposomes comprising: (i) the composition of the present invention, (ii) a liposome-forming agent, and (iii) optionally, inorganic superparamagnetic magnetites.

Moreover, the present invention also provides a pharmaceutical composition which comprises the composition of the present invention or the magnetoliposome formulation of the present invention and a pharmaceutically acceptable carrier and/or diluent. The composition of the present invention, the magnetoliposome formulation, or the pharmaceutical composition can be used as a medicament. In particular, the composition of the present invention, the magnetoliposome formulation, or the pharmaceutical composition can be used in the treatment of cancer.

Finally, the present invention also provides the use of the composition of the present invention, the magnetoliposome formulation, or the pharmaceutical composition of the present invention for the preparation of contrast agents for clinical imaging techniques. The present invention also provides the use of the composition of the present invention for (i) nucleic acid isolation; (ii) as a molecular separator; (iii) as biosensors.

### Detailed description of the invention

### Definitions

The terms *"treatment'* and *"therapy",* as used in this specification, refer to a set of hygienic, pharmacological, surgical, and/or physical protocols used with the intent to cure and/or alleviate a disease and/or symptoms with the goal of improving the state of health. The terms "*treatment*" and *"therapy"* include preventive and curative methods, since both are directed to the maintenance or reestablishment of health of an individual or animal. Regardless of the origin of the symptoms, disease and disability, the administration of a suitable medicament to alleviate and/or cure a health problem should be interpreted as a form of treatment or therapy within the context of this specification.

The term *"therapeutically effective amount*" refers to the amount of compound in a composition or formulation which has a therapeutic effect and which is able to treat a disease.

As used herein, *"pharmaceutically acceptable carrier"* or "*pharmaceutically acceptable diluent*" refers to any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are not toxic for the subject at the dosages and concentrations employed and, without limiting the scope of the present invention, include: buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the characteristics of the pharmaceutical composition.

The term "*therapeutic agent*" refers to any substance which can be used to treat and/or prevent a disease when used in therapeutically effective amounts. The therapeutic agent may be a small chemical molecule (for example, a doxorubicin, antihistamine, etc.), biological molecule (for example, a therapeutic protein), and/or nucleic acid (for example, siRNA, gRNA for CRISPR/Cas9, etc.).

The term "*chemotherapeutic agent*" refers to any drug which can be used to treat or prevent cancer. Non-limiting examples include: Actinomycin. All-trans retinoic acid, Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Mechlorethamine, Mercaptopurine, Methotrexate, Mitoxantrone, Oxaliplatin, Paclitaxel, Pemetrexed, Teniposide, Tioguanine, Topotecan, Valrubicin, Vemurafenib, Vinblastine, Vincristine, Vindesine and Vinorelbine.

The term *"cancer"* refers to a group of diseases, which can be defined as any abnormal benign or malignant new growth of tissue that possesses no physiological function and arises from uncontrolled usually rapid cell proliferation and has the potential to invade or spread to other parts of the body. The composition and the magnetoliposome formulation of the present invention can specifically recognize cancer cells by means of functionalization with a signaling substance and can further carry anti-proliferative agents to solid tumors or hematological cancers. Some non-limiting examples include: Acute granulocytic leukemia, Acute lymphocytic leukemia, Acute myelogenous leukemia, Adenocarcinoma, Adrenal cancer, Anaplastic astrocytoma, Angiosarcoma, Appendix cancer, Astrocytoma, Basal cell carcinoma, B-Cell lymphoma, Bile duct cancer, Bladder cancer, Bone cancer, Bone marrow cancer, Bowel cancer, Brain cancer, Brain stem glioma, Brain tumor, Breast cancer, Carcinoid tumors, Cervical cancer, Cholangiocarcinoma, Chondrosarcoma, Chronic lymphocytic leukemia, Chronic myelogenous leukemia, Colon cancer, Colorectal cancer, Craniopharyngioma, Cutaneous lymphoma, Cutaneous melanoma, Diffuse astrocytoma, Ductal carcinoma in situ, Endometrial cancer, Ependymoma, Epithelioid sarcoma, Esophageal cancer, Ewing sarcoma, Extrahepatic bile duct cancer, Eye cancer, Fallopian tube cancer, Fibrosarcoma, Gallbladder cancer, Gastric cancer, Gastrointestinal cancer, Gastrointestinal carcinoid cancer, Gastrointestinal stromal tumors, Germ cell tumor, Glioblastoma multiforme, Glioma, Hairy cell leukemia, Head and neck cancer, Hemangioendothelioma, Hodgkin lymphoma, Hypopharyngeal cancer, Infiltrating ductal carcinoma, Infiltrating lobular carcinoma, Inflammatory breast cancer, Intestinal Cancer, Intrahepatic bile duct cancer, Invasive/infiltrating breast cancer, Islet cell cancer, Jaw cancer, Kaposi sarcoma, Kidney cancer, Laryngeal cancer, Leiomyosarcoma, Leptomeningeal metastases, Leukemia, Lip cancer, Liposarcoma, Liver cancer, Lobular carcinoma in situ, Low-grade astrocytoma, Lung cancer, Lymph node cancer, Lymphoma, Male breast cancer, Medullary carcinoma, Medulloblastoma, Melanoma, Meningioma, Merkel cell carcinoma, Mesenchymal chondrosarcoma, Mesenchymous, Mesothelioma, Metastatic breast cancer, Metastatic melanoma, Metastatic squamous neck cancer, Mixed gliomas, Mouth cancer, Mucinous carcinoma, Mucosal melanoma, Multiple myeloma, Mycosis Fungoides, Myelodysplastic Syndrome, Nasal cavity cancer, Nasopharyngeal cancer, Neck cancer, Neuroblastoma, Neuroendocrine tumors, Non-Hodgkin lymphoma, Non-non-small cell lung cancer, Oat cell cancer, Ocular cancer, Ocular melanoma, Oligodendroglioma, Oral cancer, Oral cavity cancer, Oropharyngeal cancer, Osteogenic sarcoma, Osteosarcoma, Ovarian cancer, Ovarian epithelial cancer, Ovarian germ cell tumor, Ovarian primary peritoneal carcinoma, Sex cord stromal tumor of the ovary, Pancreatic cancer, Papillary carcinoma, Paranasal sinus cancer, Parathyroid cancer, Pelvic cancer, Penile cancer, Peripheral nerve cancer, Peritoneal cancer, Pharyngeal cancer, Pheochromocytoma, Pilocytic astrocytoma, Pineal region tumor, Pituitary gland cancer, Primary central nervous system lymphoma, Prostate cancer, Rectal cancer, Renal cell carcinoma, Renal pelvis cancer, Rhabdomyosarcoma, Salivary gland cancer, Sarcoma, Bone sarcoma, Soft tissue sarcoma, Uterine sarcoma, Sinus cancer, Skin cancer, Small cell lung cancer, Small intestine cancer, Spinal cancer, Spinal column cancer, Spinal cord cancer, Spinal tumor, Squamous cell carcinoma, Stomach cancer, Synovial sarcoma, T-cell lymphoma, Testicular cancer, Throat cancer, Thymoma/thymic carcinoma, Thyroid cancer, Tongue cancer, Tonsil cancer, Transitional cell cancer, Triple-negative breast cancer, Tubal cancer, Tubular carcinoma, Urethral cancer, Uterine adenocarcinoma, Uterine cancer, Vaginal cancer and Vulvar cancer.

The term *"substantially pure mineral phase"* refers to a mineral phase which mostly consists of a single type of mineral (≥ 95%). In this case, a substantially pure mineral phase of magnetite means that the magnetite crystals do not contain siderite. The substantially pure mineral phase of magnetite may contain small amounts of goethite (≤ 5%) if the pH is raised above 9 during the production process.

The term *"superparamagnetism"* refers to a form of magnetism which appears in small ferromagnetic or ferrimagnetic nanoparticles. Below a certain size, magnetic nanoparticles can no longer support the static walls of different magnetic domains, behaving like a giant "spin" of magnetic moment. A *"superparamagnetic"* particle can be free (thermally equilibrated) or blocked (not equilibrated). Blocking temperature (T_{B}) was determined as that at which the maximum in magnetization occurred in ZFC curves, while irreversibility temperature (Tᵢᵣᵣ) is such temperature right below the blocking of the superparamagnetic nanoparticles which are no longer thermally equilibrated.

The term *"signaling substance"* refers to any molecule which is able to specifically bind to a given substance. Non-limiting examples include antibodies, affibodies, aptamers, etc. In a preferred embodiment, the signaling substance is a monoclonal antibody.

The term *"inorganic magnetite nanoparticles"* or MNPs refers to any magnetite nanoparticle which is obtained or obtainable through chemical synthesis methods in the absence of any biological agent and/or product.

The term *"MamC"* refers to a full length protein which is derived from the mamC gene (NCBI Database, gene accession number ABK44766.1, protein accession Mmc1_2265). The term *"MamC"* also includes functional fragments and variants of the protein derived from the mamC gene which may be expressed in biological systems or synthesized. Functional MamC fragments have been described previously (Nudelman *et al.,* 2016; Nudelman *et al.,* 2018; patent WO 2017153996), in particular, functional fragments containing the MamM region-interaction region (MamC-MIL) (Nudelman *et al.,* 2016). The functional fragments may be fused to another protein such as MBP.

The term *"Mms6"* refers to a full length protein which is derived from the *mms6* gene (NCBI Database, gene accession number ABK44766.1, protein accession Mmc1_2275). The term *"Mms6"* also includes functional fragments and variants of the protein derived from the *mms6* gene which may be expressed in biological systems or synthesized.

The term *"Mms7"* refers to a full length protein which is derived from the *mms7* gene (UniProtKB reference number Q2W8R9). The term *"Mms7"* also incldues functional fragments and variants of the protein derived from the *mms7* genewhich may be expressed in biological systems or synthesized.

The term "*functional variant*" refers to any variant or mutant that has a certain percentage (degree) of homology with the protein and maintains the function of the protein. In a preferred embodiment, a functional variant has a degree of homology with the protein greater than 75%. Preferably, the degrees of homology with the original protein are 80, 85, 90, 95, 98 or 99%. More preferably, the degree of homology with the original protein is 95%.

The degree of identity between protein sequences can be determined through conventional methods. For example, by using standard sequence alignment logarithms known in the state of the art such as BLAST (Altschul et al. 1990 J Mol Biol. 215 (3): 403-10) or CLUSTAL O (1.2.1). In a preferred embodiment, the degree of homology is determined using BLAST or CLUSTAL O.

### Method of the invention

In a first aspect, the present invention provides a method for producing a composition comprising a substantially pure mineral phase of superparamagnetic magnetite, said method comprising the following steps: (**a**) preparing a carbonate solution, (**b**) adding FeCl₃ to the carbonate solution, (**c**) adding MamC and, optionally, Mms6 to the solution obtained in step (b), (**d**) incubating the solution obtained in step (c) for at least 30 minutes, (**e**) adding Fe(ClO₄)₂ to the solution obtained in step (d), and (**f**) adjusting the pH of the solution obtained in step (e) to pH 9 using a base; wherein, the method is performed at 25°C and 1 atmosphere of pressure. All the solutions used are previously deoxygenated. For a better result, the method of the present invention is performed under anoxic conditions, i.e., less than 40 ppb oxygen in the solution.

The present invention provides a sequence of steps, which are essential for producing a composition that is free of any detectable level of siderite. The sequence of steps provided allows the highly hydrophobic MamC protein to remain folded and functional while keeping the solution supersaturated for magnetite in such a way that the precipitation of magnetite is kinetically favored with respect to that of siderite. Thus, obtaining the correct sequence required the careful balance of these three aspects.

In the best conditions, Mms7 is also added to the solution obtained in step (b) in step (c) of the method.

In the best conditions, the concentration of the protein(s) added in step (c) must be at least 2 mg/mL. In the best conditions, the concentration of the MamC solution that is added to the solution in step (c) is 2-5 mg/mL. Higher concentrations may induce aggregation and thus, prevent or reduce the efficiency of MamC mediation in magnetite biomineralization process. Lower concentrations will result in magnetite crystals of fairly small size (< 5 nm) due to the prevalence of the effect of the TRIS buffer on the biomineralization process. This embodiment does not refer to the final concentration of the protein solution. This embodiment refers to the concentration of the stock solution which is added into the solution obtained after step (b).

In the best conditions, the solution is incubated for at least 1 hour in step (d).

In the best conditions, the carbonate solution comprises NaHCO₃ and/or Na₂CO₃ and, optionally, the base is NaOH.

In the best conditions, the final concentration of the solution obtained in step (f) is 3.5 mM NaHCO₃, 3.5 mM Na₂CO₃, 2.78 mM Fe(ClO₄)₂, 5.56 mM FeCl₃ and a variable amount of MamC and, optionally, Mms6. Preferably, the final concentration of MamC is 10 µg/mL and, if Mms6 is included, the final concentration of MamC is at least 5 µg/mL and the final concentration of Mms6 is 10 µg/mL.

### Composition of the invention

In a second aspect, the present invention provides a composition obtained or obtainable through the methods of the present invention. In a third aspect, the present invention provides a composition comprising: (i) a substantially pure mineral phase of superparamagnetic magnetite, (ii) the MamC protein, and (iii) optionally, the Mms6 protein; wherein, at least components (i) and (ii) form superparamagnetic magnetic nanoparticles containing up to 5 wt% of MamC, with a mean particle size between 30 and 120 nm.

Preferably, the MamC-mediated BMNPs are composed of ∼95 wt% of magnetite and ∼5 wt% of MamC, with an isoelectric point of ∼4.4, a specific surface area of ∼ 90 m²/g, a blocking temperature of ∼145 K, and an irreversibility temperature of -292 K.

In the best conditions, all of the following aspects of the present invention are applicable to the second and third aspects of the present invention. It is apparent that the second and third aspects of the present invention may refer to the same composition.

In the best conditions, there are no detectable levels of siderite in the composition. In an even more preferred manner, the levels of goethite are ≤ 5% of the total solid.

In the best conditions, the mean particle size of the magnetic nanoparticles is 30-50 nm. Preferably, the mean particle size of the magnetic nanoparticles is 30-40 nm. In the best conditions, the mean particle size is determined using transmission electron microscopy. In the examples of the present invention, the sizes were obtained by measuring the size of more than 1000 crystals in each transmission electron microscopy image.

In the best conditions, the wt% of MamC in the BMNPs is 2-20 wt%. Preferably, the wt% of MamC in the BMNPs is 2-10 wt%. In the best conditions, the wt% of MamC in the BMNPs is determined by thermogravimetric analysis. In the examples of the present invention, the wt% of MamC in the BMNPs is 5 wt%.

The composition may further comprise other proteins involved in the formation of magnetite in the magnetosomes of bacteria and/or other proteins with acidic domains capable of binding iron and/or those with such a structure that could provide a template for magnetite nucleation and growth. Non-limiting examples of such proteins include Mms6, Mms7, MmsF/MamF and their homologous proteins in different magnetotactic bacteria. In the best conditions, the composition further comprises Mms7.

In the best conditions, the isoelectric point of the BMNPs is 3-7. Preferably, the isoelectric point of the BMNPs is 3-5. In the best conditions, the isoelectric point of the BMNPs is calculated from measurements of the electrophoretic mobility. In the examples of the present invention, the isoelectric point of the BMNPs is 4.4.

In the best conditions, the specific surface area of the BMNPs is 30-120 m²/g. Preferably, the specific surface area of the BMNPs is 50-100 m²/g. In the best conditions, the specific surface area of the BMNPs is determined by BET. In the examples of the present invention, the specific surface area of the BMNPs is 97 m²/g.

The nanoparticles formed using the MamC protein exhibit a large magnetization per particle at room temperature that is equal to or higher than that of the nanoparticles obtained through inorganic methods or through the use of Mms6 protein alone. Thus, in the best conditions, the magnetization of the BMNPs is 40-70 emu/g at 300K when an external magnetic field of 500 Oe is applied. Preferably, the magnetization of the BMNPs is 55-65 emu/g at 300K when an external magnetic field of 500 Oe is applied. In optimal conditions, the magnetization of the BMNPs is 55 emu/g (61 emu/g when the amount of MamC in the crystal is taken into account) at 300K when an external magnetic field of 500 Oe is applied.

In the best conditions, the blocking temperature of the BMNPs is at least 100 K when an external magnetic field of 500 Oe is applied. Preferably, the blocking temperature is at least 120 K when an external magnetic field of 500 Oe is applied. In optiomal conditions, the blocking temperature is at least 130 K when an external magnetic field of 500 Oe is applied.

In the best conditions, the irreversibility temperature of the BMNPs is at least 200 K when an external magnetic field of 500 Oe is applied. Preferably, the irreversibility temperature is at least 250 K when an external magnetic field of 500 Oe is applied. In optimal conditons, the irreversibility temperature is at least 280 K when an external magnetic field of 500 Oe is applied.

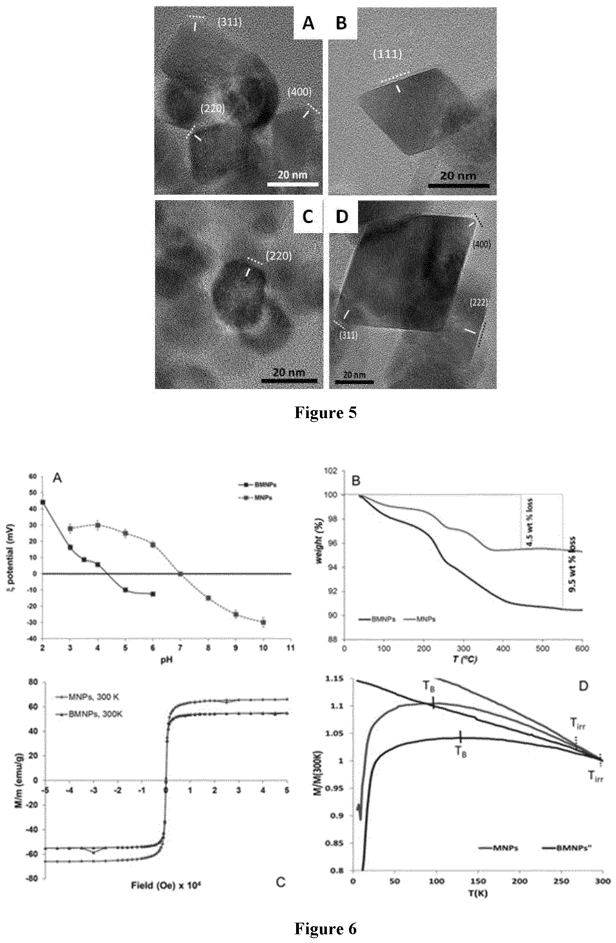

An advantage of biomimetic nanoparticles in comparison to inorganic ones is the fact the proteins change the surface properties, since MamC is adsorbed and/or incorporated at the crystal surface up to 5 wt% (Figure 6B). In particular, for example, MamC-magnetites present zero point charge at a pH of about 4 (Figure 6A). Therefore, they are strongly charged at physiological pH (pH = 7.4) and can adsorb high amounts of polar drugs. Said adsorption is stable at physiological pH and negligible drug release occurs at this pH. However, when the functionalized nanoparticle is exposed to acidic pH values (such as those that exist in the tumor microenvironment or within the cell lysosome), the particle loses charge and releases the drug. On the contrary, inorganic magnetite nanoparticles (MNPs) have a zero point charge at a pH of about 7.4, and, therefore, are neutral or slightly charged at physiological pH. Thus, low adsorption and high drug release are expected at physiological pH values. In order to prevent this, the nanoparticle must be covered by a molecular coating that allows for a stable functionalization. This step is not necessary in the biomimetic nanoparticles that are the object of this patent.

In the best conditions, the magnetic nanoparticles are functionalized with a therapeutic agent. The therapeutic agent can be any agent which has a therapeutic effect when administered in a therapeutically effective amount. Preferably, the therapeutic agent is a chemotherapeutic agent or a nucleic acid molecule. Nucleic acid molecules include both RNA and DNA. The RNA can be a siRNA, miRNA or gRNA (gRNA for use in CRISPR/Cas9 gene editing approaches).

In the best conditions, the magnetic nanoparticles are functionalized with a chemotherapeutic agent. In the best conditions, the chemotherapeutic agent is polar. Preferably, the chemotherapeutic agent is doxorubicin.

In the best conditions, the magnetic nanoparticles are functionalized with a signaling substance. Preferably, the signaling substance is a ligand for a growth factor receptor (such as a growth factor), an antibody or an aptamer. More preferably, the signaling substance is a monoclonal antibody.

In the best conditions, the magnetic nanoparticles are functionalized with a chemotherapeutic agent and a signaling substance. Preferably, the chemotherapeutic agent is doxorubicin and the signaling substance is a monoclonal antibody.

In the best conditions, the composition consists of: (i) a substantially pure mineral phase of superparamagnetic magnetite, (ii) MamC, and (iii) optionally, Mms6 and/or Mms7; wherein, at least components (i) and (ii) form superparamagnetic magnetic nanoparticles containing up to 5 wt% of MamC (MamC-mediated BMNPs are thus composed of ∼95 wt% of magnetite and ∼5 wt% of MamC), with a mean particle size between 30 and 120 nm, an isoelectric point of ∼4.4, a specific surface area of ∼ 90 m²/g, a blocking temperature of ∼145 K, and an irreversibility temperature of ∼292 K.

In the best conditions, the composition is not derived, obtained or obtainable from a magnetosome. More preferably, the composition is not obtained from a magnetosome. To clarify, in this condition, while the proteins used in the composition may be derived from a magnetosome, the BMNPs are obtained using the *in vitro* precipitation approach disclosed in the present invention. In other words, the superparamagnetic magnetic nanoparticles are biomimetic superparamagnetic magnetic nanoparticles (BMNPs).

In the best conditions, the superparamagnetic magnetic nanoparticles are biomimetic superparamagnetic magnetic nanoparticles.

The term *"magnetosome"* refers to both natural magnetosomes present in magnetotactic bacteria as well as recombinant magnetosomes or magnetosome-like structures that are produced by a host which does not normally contain magnetosomes or magnetosome-like structures.

### Magnetoliposome formulation

In a fourth aspect, the present invention provides a magnetoliposome formulation comprising: (i) the composition of the present invention, (ii) a liposome-forming agent; and (iii) optionally, inorganic superparamagnetic magnetite nanoparticles (MNPs).

The liposome-forming agent in the magnetoliposome formulation is preferably a hydrogenated, partially hydrogenated or non-hydrogenated phospholipid. The phospholipid used can be or comprise, for example: phosphatidylcholine, phosphatidylserine and phosphatidyl-inositol. Most typical is phosphatidylcholine, which can be synthesized or isolated from a variety of natural sources. Besides phosphatidylcholine, there are other phospholipids which can also be used in the formulation, either as liposome-forming agents or as additional components. These phospholipids are: dicetyl phosphate (DCP), dimyristoyl phosphatidylcholine (DMPC), dimyristoyl phosphatidylglycerol (DMPG), dioleoyl phosphatidylcholine (DOPc), dipalmitoyl phosphatidylcholine (DPPC), dipalmitoyl phosphatidylglycerol (DPPG), phosphatidylcholine (PC) and/or phosphatidylserine (PS), whereby the lipid involved in the formulation may be hydrogenated, partially hydrogenated or non-hydrogenated. The magnetoliposomes can be formed using conventional auxiliary lipids by measn of techniques well-known by the person skilled in the art, such as those described in patent application ES2231037-A1.

Although liposomes have been used to coat inorganic magnetite nanoparticles, no biomimetic nanoparticles had previously been encapsulated in liposomes. Since the surface properties of both particles are very different, the process for stabilizing respective types of particles before encapsulating them in a liposome is very different. Encapsulating the nanoparticles without prior stabilization would result in the agglomeration of said particles. Agglomerated nanoparticles would not be useful for nanotechnology applications. Thus, the process for obtaining magnetoliposomes comprising the use of biomimetic magnetite nanoparticles is not obvious or straightforward.

Superparamagnetic nanoparticles such as inorganic magnetite nanoparticles (MNPs) can be especially useful contrast agents for magnetic resonance imaging and hyperthermia treatments derived from the temperature increase caused by the rotation of the magnetic nanoparticles induced by an alternating magnetic field or by radiation. Thus, a magnetoliposome having MNPs that are larger and smaller in size may be able to utilize the advantages of both types of nanoparticles. In this aspect, in a preferred embodiment of the invention, the magnetoliposome formulation further comprises MNPs.

In a preferred embodiment of the invention, the magnetoliposomes of the magnetoliposome formulation are functionalized with therapeutic agents and/or a targeting substance. The magnetoliposomes can be functionalized through any common method known in the art. For example, the magnetoliposomes can be functionalized using the methods described by Torchilinet *et al.,* 2001. The therapeutic agent can be any agent which has a therapeutic effect when administered in a therapeutically effective amount. In a preferred embodiment of the invention, the therapeutic agent is a chemotherapeutic agent and the targeting substance is an antibody. More preferably, the antibody is a monoclonal antibody.

### Pharmaceutical composition

In a fifth aspect, the present invention provides a pharmaceutical composition which comprises the composition of the present invention or the magnetoliposome formulation of the present invention and a pharmaceutically acceptable vehicle and/or diluent.

In a preferred embodiment of the invention, the pharmaceutical composition may comprise one or more solution(s) which are suitable for intravenous, intraarterial, intramuscular and/or subcutaneous administration. In another embodiment, the pharmaceutical composition may comprise one or more solution(s) which are suitable for sublingual, buccal and/or inhalation-mediated administration routes. In an alternative embodiment, the pharmaceutical composition may comprise one or more aerosol(s) which are suitable for inhalation-mediated administration.

In a preferred embodiment of the invention, the pharmaceutical composition may comprise one or more cream(s) and/or ointment(s) which are suitable for topical administration. In a preferred embodiment of the invention, the pharmaceutical composition may comprise one or more suppositories which are suitable for rectal or vaginal administration. In this embodiment, the composition may be used in order to achieve a loco-regional effect.

### Treatment of diseases

In a sixth aspect, the present invention provides the composition of the present invention, the magnetoliposome formulation of the present invention, or the pharmaceutical composition of the present invention for use as a medicament.

In a seventh aspect, the present invention provides the composition of the present invention, the magnetoliposome formulation of the present invention, or the pharmaceutical composition of the present invention for use in the treatment of cancer.

In a preferred embodiment, the cancer is selected from the group consisting of Acute granulocytic leukemia, Acute lymphocytic leukemia, Acute myelogenous leukemia, Adenocarcinoma, Adrenal cancer, Anaplastic astrocytoma, Angiosarcoma, Appendix cancer, Astrocytoma, Basal cell carcinoma, B-Cell lymphoma, Bile duct cancer, Bladder cancer, Bone cancer, Bone marrow cancer, Bowel cancer, Brain cancer, Brain stem glioma, Brain tumor, Breast cancer, Carcinoid tumors, Cervical cancer, Cholangiocarcinoma, Chondrosarcoma, Chronic lymphocytic leukemia, Chronic myelogenous leukemia, Colon cancer, Colorectal cancer, Craniopharyngioma, Cutaneous lymphoma, Cutaneous melanoma, Diffuse astrocytoma, Ductal carcinoma in situ, Endometrial cancer, Ependymoma, Epithelioid sarcoma, Esophageal cancer, Ewing sarcoma, Extrahepatic bile duct cancer, Eye cancer, Fallopian tube cancer, Fibrosarcoma, Gallbladder cancer, Gastric cancer, Gastrointestinal cancer, Gastrointestinal carcinoid cancer, Gastrointestinal stromal tumors, Germ cell tumor, Glioblastoma multiforme, Glioma, Hairy cell leukemia, Head and neck cancer, Hemangioendothelioma, Hodgkin lymphoma, Hypopharyngeal cancer, Infiltrating ductal carcinoma, Infiltrating lobular carcinoma, Inflammatory breast cancer, Intestinal Cancer, Intrahepatic bile duct cancer, Invasive/infiltrating breast cancer, Islet cell cancer, Jaw cancer, Kaposi sarcoma, Kidney cancer, Laryngeal cancer, Leiomyosarcoma, Leptomeningeal metastases, Leukemia, Lip cancer, Liposarcoma, Liver cancer, Lobular carcinoma in situ, Low-grade astrocytoma, Lung cancer, Lymph node cancer, Lymphoma, Male breast cancer, Medullary carcinoma, Medulloblastoma, Melanoma, Meningioma, Merkel cell carcinoma, Mesenchymal chondrosarcoma, Mesenchymous, Mesothelioma, Metastatic breast cancer, Metastatic melanoma, Metastatic squamous neck cancer, Mixed gliomas, Mouth cancer, Mucinous carcinoma, Mucosal melanoma, Multiple myeloma, Mycosis Fungoides, Myelodysplastic Syndrome, Nasal cavity cancer, Nasopharyngeal cancer, Neck cancer, Neuroblastoma, Neuroendocrine tumors, Non-Hodgkin lymphoma, Non-small cell lung cancer, Oat cell cancer, Ocular cancer, Ocular melanoma, Oligodendroglioma, Oral cancer, Oral cavity cancer, Oropharyngeal cancer, Osteogenic sarcoma, Osteosarcoma, Ovarian cancer, Ovarian epithelial cancer, Ovarian germ cell tumor, Ovarian primary peritoneal carcinoma, Sex cord stromal tumor of the ovary, Pancreatic cancer, Papillary carcinoma, Paranasal sinus cancer, Parathyroid cancer, Pelvic cancer, Penile cancer, Peripheral nerve cancer, Peritoneal cancer, Pharyngeal cancer, Pheochromocytoma, Pilocytic astrocytoma, Pineal region tumor, Pituitary gland cancer, Primary central nervous system lymphoma, Prostate cancer, Rectal cancer, Renal cell carcinoma, Renal pelvis cancer, Rhabdomyosarcoma, Salivary gland cancer, Sarcoma, Bone sarcoma, Soft tissue sarcoma, Uterine sarcoma, Sinus cancer, Skin cancer, Small cell lung cancer, Small intestine cancer, Spinal cancer, Spinal column cancer, Spinal cord cancer, Spinal tumor, Squamous cell carcinoma, Stomach cancer, Synovial sarcoma, T-cell lymphoma, Testicular cancer, Throat cancer, Thymoma/thymic carcinoma, Thyroid cancer, Tongue cancer, Tonsil cancer, Transitional cell cancer, Triple-negative breast cancer, Tubal cancer, Tubular carcinoma, Urethral cancer, Uterine adenocarcinoma, Uterine cancer, Vaginal cancer, acute lymphocytic leukemia, acute myelogenous leukemia, thymomas, transitional cell bladder cancer, Wilms' tumor, Waldenstrom macroglobulinemia and Vulvar cancer. Preferably, the cancer is selected from the group consisting of acute lymphocytic leukemia, acute myelogenous leukemia, bone sarcoma, breast cancer, endometrial cancer, gastric cancer, head and neck cancer, Hodgkin lymphoma, Non-Hodgkin lymphoma, liver cancer, kidney cancer, multiple myeloma, neuroblastoma, ovarian cancer, small cell lung cancer, soft tissue sarcoma, thymomas, thyroid cancer, transitional cell bladder cancer, uterine sarcoma, Wilms' tumor and Waldenstrom macroglobulinemia.

In a preferred embodiment, the present invention provides a composition comprising: (i) a substantially pure mineral phase of biomimetic superparamagnetic magnetite, (ii) MamC, and (iii) optionally, Mms6; wherein, at least components (i) and (ii) form superparamagnetic magnetic nanoparticles containing up to 5 wt% of MamC (MamC-mediated biomimetic nanoparticles are thus composed of ∼95 wt% of magnetite and ∼5 wt% of MamC), with a mean particle size between 30 and 120 nm, an isoelectric point of ∼4.4, a specific surface area of ∼ 90 m²/g, a blocking temperature of ∼145 K, and an irreversibility temperature of ∼292 K and, wherein the magnetic nanoparticles are functionalized with a chemotherapeutic agent; and a pharmaceutically acceptable carrier and/or diluent.

Preferably, the chemotherapeutic agent is doxorubicin and the cancer is selected from the group consisting of acute lymphocytic leukemia, acute myelogenous leukemia, bone sarcoma, breast cancer, endometrial cancer, gastric cancer, head and neck cancer, Hodgkin lymphoma, Non-Hodgkin lymphoma, liver cancer, kidney cancer, multiple myeloma, neuroblastoma, ovarian cancer, small cell lung cancer, soft tissue sarcoma, thymomas, thyroid cancer, transitional cell bladder cancer, uterine sarcoma, Wilms' tumor and Waldenstrom macroglobulinemia.

In a preferred embodiment, the treatment of cancer involves the use of a hyperthermia treatment. In such a treatment, an alternating magnetic field or radiation is used to rotate the magnetic nanoparticles. The magnetic nanoparticles then increase the surrounding temperature in response to the energy generated through the rotations. If the magnetite nanoparticles are localized to the cancer cells, they may cause the cancer cells to die due to the increase in heat.

### Uses

In an eighth aspect, the present invention provides the use of the composition of the present invention, the magnetoliposome formulation of the present invention, or the pharmaceutical composition of the present invention for the preparation of a contrast agent for use in clinical imaging techniques such as magnetic resonance imaging. In a ninth aspect, the present invention provides the use of the composition of the present invention for nucleic acid isolation and/or purification.

Other uses of the composition of the present invention, the magnetoliposome formulation of the present invention, or the pharmaceutical composition of the present invention, are also envisaged and also form part of the invention. These uses include the use of the composition of the present invention, the magnetoliposome formulation of the present invention, or the pharmaceutical composition of the present invention as a molecular separator (e.g. functionalizing the nanoparticles with antibodies to capture a specific molecule and then separating said molecule using a magnetic force) and the use of the composition of the present invention, the magnetoliposome formulation of the present invention, or the pharmaceutical composition of the present invention as a biosensor. The biosensor can be for use in a clinical setting or environmental setting.

### EMBODIMENTS

### Example 1: Multiple sequence alignment of MamC and Mms6

A multiple sequence alignment of MamC and Mms6 from MC-1 with other homologous proteins from other magnetotactic bacteria showed some similarities in their C-terminal domain and also in the loop within the two transmembrane domains. This loop is rich in amino acids (aspartate and glutamate) and in amino acids containing hydroxyl groups (tyrosine, threonine and serine) which can bind metal cations. (Figures 1 and 2).

### Example 2: Cloning and expression of MamC and Mms6

MamC cloning, expression and purification was carried out as described in Valverde-Tercedor *et al.,* 2015. Briefly, the mamC gene(NCBI Database, gene accession ABK44766.1, protein accession Mmc1_2265) was amplified by polymerase chain reaction and cloned into a pTrcHis-TOPO vector (Life Technologies: Invitrogen, Grand Island, NY) so that the recombinant MamC protein is expressed with an N-terminal hexahistidine tag. The recombinant vector was transformed into an *Escherichia coli* TOP10 strain (Life Technologies: Invitrogen) and was verified by dideoxynucleotide sequencing using an ABI model 3100 sequencer (Life Technologies: Applied Biosystems).

For the expression and purification of the MamC protein, transformed *E. coli* TOP10 cells were incubated at 37°C and protein expression was induced with isopropyl β-D-1-thiogalactopyranoside (IPTG). Cells were harvested by centrifugation, resuspended in buffer A (20 mM sodium phosphate buffer, 500 mM NaCl, 6 M guanidine, pH 8.0) and disrupted by sonication. The soluble fraction was separated by centrifugation and loaded onto a HiTrap chelating HP column (GE Healthcare) previously equilibrated with buffer B (20 mM sodium phosphate buffer, 500 mM NaCl, 8 M urea, pH 8.0), using an ÄKTA Prime Plus FPLC system (GE Healthcare). The column was then washed with buffer B, followed by buffer B adjusted to pH 6. Finally, the protein was eluted with buffer B adjusted to pH 4. The eluate was dialyzed overnight at 4°C against buffer C (50 mM Tris buffer, 150 mM NaCl, 6 M urea, pH 8.5). To reduce the concentration of urea, the dialysis buffer was diluted stepwise 1:2 (four times) with fresh buffer C without urea (named buffer D) and dialyzed for another 2-4 h after each dilution step except for the last step that was dialyzed overnight.

The *mms6* gene (NCBI Database, gene accession ABK44776.1, protein accession Mmc1_2275) was amplified by polymerase chain reaction using the specific primers: f6 (SEQ ID NO: 1, 5'-ATGCCTGTTGCTGTACCAAATAAAGC-3') and r6 (SEQ ID NO: 2, 5'-TCAGCTAATGGCCTCTTCCAATTC-3'). As in the case of *mamC*, the amplified *mms6* gene was cloned into a pTrcHis-TOPO vector. The recombinant vector was also used to transform an *Escherichia coli* TOP10 strain and verified by dideoxynucleotide sequencing.

The expression and purification of the Mms6 protein was carried out following the same protocol as described above for the purification of MamC, but using 1 mM IPTG instead. Cells were harvested by centrifugation (4508 g, 10 min, 4°C), resuspended in 20 mM sodium phosphate buffer (pH 7.4) supplemented with 0.5 mg/mL lysozyme and 5% sodium lauroyl sarcosinate (sarkosyl) and disrupted by sonication. The soluble fraction was separated by centrifugation (15151 g, 40 min, 4°C) and loaded onto a HiTrap chelating HP column (GE Healthcare) using an ÄKTA Prime Plus FPLC system (GE Healthcare). The column was previously equilibrated with 20 mM sodium phosphate buffer (pH 7.4) supplemented with 20 mM imidazole and TRITON X-100 at 1.3 x the critical micelle concentration (CMC) to reduce protein aggregation and to improve protein stability. The elution of Mms6 (2 mL/min) was performed by applying a continuous imidazole gradient from 20 to 500 mM. Fractions were collected and analyzed by 12% SDS-PAGE electrophoresis. Fractions containing Mms6 protein were subjected to an additional chromatographic step in a C4 hydrophobicity column (Jupiter® 5 µm C4 300 Å, LC Column 150 x 4.6 mm) using an HPLC system (Agilent 1100) to remove minor contaminants, *E. coli* proteins and nucleic acids. In this case, the elution of Mms6 protein (0.5 mL/min) occurred by applying a continuous organic solvent (trifluoroacetic acid and acetonitrile) gradient in water because of the high hydrophobicity of Mms6. The purity of the Mms6 protein was tested by Coomassie-stained 12% SDS-PAGE. Protein concentration was determined using a Bradford protein assay (Bradford, 1976) and using a NanoDrop 2000 UV-Vis spectrophotometer (Thermo Scientific), using the corresponding molar extinction coefficient at 280 nm (17085 M⁻¹ cm⁻¹).

As a control experiment, TOP10 competent cells were also transformed with pTrcHis-TOPO that did not contain the genes of interest. The purification protocol of MamC and Mms6 was followed with those transformed bacteria and their corresponding elution fractions were used for magnetite precipitation (control) experiments.

Figure 1 shows an SDS-PAGE gel of the purified MamC and Mms6 proteins.

### Example 3: Magnetite biomineralization

Deoxygenated solutions of NaHCO₃/Na₂CO₃ (0.15 M/ 0.15 M), FeCl₃ (1 M), Fe(ClO₄)₂ (0.5 M), and NaOH (5 M) were prepared by using oxygen-free deoxygenated Milli-Q water (ultrapure or "Type 1" as defined by various authorities, e.g. ISO 3696) according to the following procedure:
(1) To prepare the anaerobic solutions:
   a. Boil the Milli-Q water in an Erlenmeyer flask in the presence of bubbling rocks until big bubbles form and escape out of the solution
   b. Once boiled, place the flask in an ice bath and immediately cover it and let it bubble with O₂-free N₂ for an hour/L.
   c. Once bubbled, place the water inside the anaerobic Coy Chamber and prepare the solutions inside said chamber.
(2) To prepare the anaerobic protein solutions from a protein stock solution with a protein concentration higher than 2 mg/mL:
   a. Cover the protein solution with a rubber septum and bubble with O₂-free N₂.
   b. Once bubbled, place the solution inside the anaerobic Coy Chamber.
(3) To prepare the final mixture for magnetite precipitation (preferably 60 mL in 100 mL glass bottles):
   a. Add the pertinent volume of the NaHCO₃ and Na₂CO₃ solutions to ensure a final concentration in the reaction mixture of 3.5 mM each.
   b. Add the total pertinent volume of the Milli-Q water.
   c. Add the pertinent volume of the FeCl₃ solution to ensure a final concentration in the reaction mixture of 5.56 mM.
   d. Add the protein(s) if needed.
   e. Let it react for one hour.
   f. Add the pertinent volume of the Fe(ClO₄)₂ solution to ensure a final concentration in the reaction mixture of 2.78 mM.
   g. While stirring, add drops of the solution of NaOH to raise the pH value to a pH of 9.
   h. Close the bottles with a rubber septum and seal them and let them react inside the anaerobic Coy chamber for 30 days.

The COY chamber was filled with 4% H₂ in N₂ to avoid potential oxidation. Magnetite precipitation was carried out in free-drift experiments and held at 25°C and 1 atm total pressure following the protocol described by the authors inside the anaerobic chamber. The final reaction mixture from which magnetite precipitated contained 3.5 mM NaHCO₃/3.5 mM Na₂CO₃, 2.78 mM Fe(ClO₄)₂ and 5.56 mM FeCl₃, and had a pH = 9. MamC and/or Mms6 were added to this reaction mixture at concentrations ranging from 0 to 10 µg/mL. Specifically, nineteen magnetite coprecipitation experiments were carried out under the following conditions (three replicates per condition): (1) sixteen experiments performed by adding MamC and Mms6 to the reaction solution at protein concentrations of 0, 2.5, 5, 10 µg/mL and MamC/Mms6 and Mms6/MamC ratios ranging from 0 to 4, here referred to as MamC-, Mms6-, MamC-Mms6-bearing experiments; (2) one experiment performed by adding to the reaction mixture the "contaminant" proteins purified from cells transformed with the "empty" pTrcHis-TOPO, here referred to as empty-vector experiment; (3) two experiments carried out by adding to the reaction mixture the buffer in which each one of the proteins was stored (50 mM Tris and 150 mM NaCl (here referred to as MamC-buffer experiment)) and 1.3 CMC TRITON X100 in water (here referred to as Mms6-buffer experiment); (4) one inorganic experiment in which no proteins and/or buffer were added to the reaction mixture.

Each experiment was allowed to proceed inside the anaerobic chamber for 30 days, after which the precipitated product was harvested. The solids were concentrated in tubes with a magnet and the supernatant (that looked completely clear) was discarded. Then the precipitates were washed with oxygen-free deoxygenated Milli-Q water two times and a last wash was performed with absolute ethanol (5 mL in each reaction). Between washes, each reaction flask was vigorously shaken for several seconds, the precipitate was magnetically concentrated and the liquid removed. After the last ethanol washing the precipitate was concentrated in 1-2 mL of ethanol, hermetically sealed and stored at - 20°C until analyzed.

### Example 4: Identification of the precipitates

Powder samples of the precipitates were analyzed with an Xpert Pro X-ray diffractometer (PANalytical; The Netherlands) using Cu Kα radiation, with the scan range set from 20 to 60° in 2θ (0.01°/step; 3 s per step). Identification of the precipitates was performed using XPowder software (Martin Ramos, 2004).

The solids formed in all the biomineralization experiments (with and without the proteins) were identified as magnetite using X-ray powder diffraction (XRD). The solids contained no detectable levels of siderite.

### Example 5: Size and morphology of the particles

The morphology and size of the magnetite nanoparticles collected in those experiments were studied by Transmission Electron Microscopy (TEM) using a Carl Zeiss LIBRA 120 PLUS SMT microscope. Magnetic nanoparticles were embedded in Embed 812 resin. Ultrathin sections (50-70 nm) were prepared using a Reichert Ultracut S microtome (Leica Microsystems GmbH, Wetzlar, Germany) after which the sections were deposited onto copper grids. The size of the crystals was measured using the ImageJ 1.47 program, and size distribution curves were determined from those measurements by using Origin pro 9. To ensure reproducibility of results, particle sizes were measured on multiple micrographs with an excess of 1000 nanoparticles measured for each experiment. In addition, statistical significance of the results obtained was tested using the Tukey test with a fixed value of α<0.05. High Resolution TEM (HRTEM) was also performed using a FEI TITAN G2 80-300. The selected area electron diffraction (SAED) patterns were collected using a 10 µm aperture. D-spacings were measured using HRTEM images and the crystallographic direction was determined by using magnetite data on the RRUFF Project website (http://rruff.info/ams/amcsd.php).

TEM analysis of the magnetite particles produced in the MamC-buffer experiment, Mms6-buffer experiment, empty vector experiment and Mms6-mediated magnetites show similar crystal sizes (16 ± 6 nm) as those of magnetites collected from the inorganic control experiment. Moreover, no differences in morphology were observed either, since all the particles were poorly faceted. Therefore, the potential effect on crystal size and/or morphology observed on the magnetites collected from the remaining experiments should be solely attributed to the proteins involved.

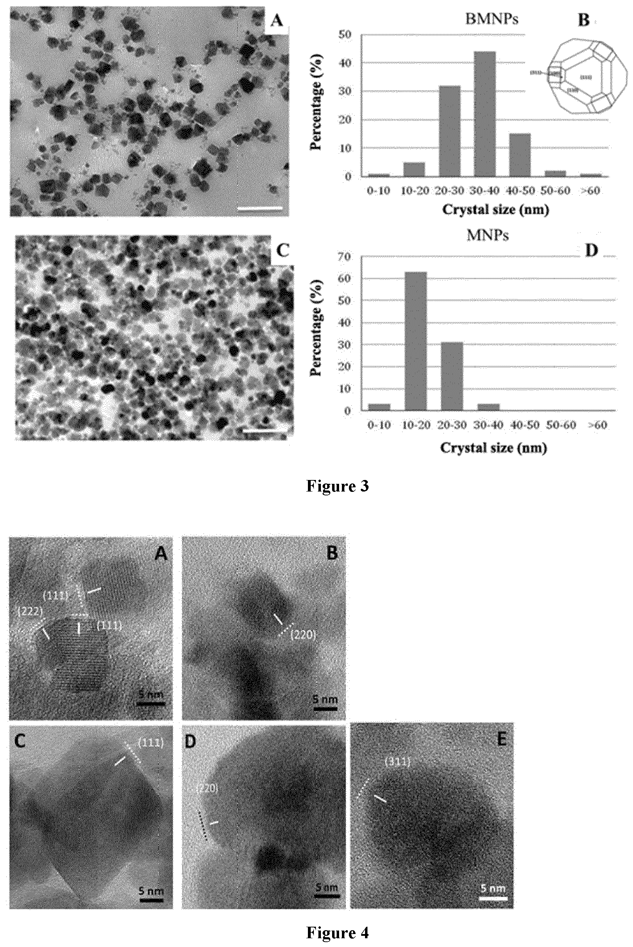

TEM images of the Mms6-mediated magnetites show differences in size and shape with respect to the inorganic control experiments depending on the concentration of Mms6 in solution. At an Mms6 concentration of 2.5 µg/mL, non-faceted crystals of 17 ± 7 nm were formed. However at Mms6 concentrations of 5 and 10 µg/mL, the magnetite crystals had more uniform polyhedral morphologies with well-faceted faces and were slightly larger in size (23 ± 9 and 22 ± 8 nm, respectively) when compared to magnetites obtained from the inorganic control (MNPs, Figure 3). The size and shape of MamC-mediated magnetite particles (BMNPs) also depended on the protein concentration. BMNP crystals formed in the presence of 2.5 µg/mL and 5 µg/mL of MamC were rounded and had sizes of 20 ± 6 nm and 22 ± 7 nm, respectively. At 10 µg/mL of MamC, the magnetite crystals displayed well-developed crystal faces with rhombic, rectangular, and square two-dimensional morphologies and sizes of 37 ± 12 nm (Figure 3).

When both MamC and Mms6 were present in the reaction solution, cumulative effects from both proteins were observed, since magnetite crystals collected from these experiments displayed better faceted morphologies and/or larger sizes compared, not only to crystals from the inorganic control experiment, but also to crystals collected from experiments in which only one of the proteins were present. The effect of the protein mixture on the magnetite crystals depended on the concentration of the two proteins and the protein ratio. At low concentrations of Mms6 (2.5 µg/mL), the size of the crystals increased with the concentration of MamC up to [MamC] = 5 µg/mL. This trend is identical to that observed at the highest concentration of Mms6 (10 µg/mL). However, at [Mms6] = 5 µg/mL no change in the size of the crystals was observed independently of the concentration of MamC in the solution. The same output is observed when the size of the crystals is represented against the MamC/Mms6 ratio, where the larger average size of the crystals (31 ± 10 nm) is obtained at a MamC/Mms6 ratio equal to 0.64 and a MamC concentration of 5 µg/mL (0.28 µM) and Mms6 concentration of 10 µg/mL (0.44 µM) (Figure 3). Interestingly, at higher MamC/Mms6 ratios, the crystal size decreases and this decrease was found to be statistically significant.

HRTEM images show that crystals obtained from the inorganic control experiments have a square and some rhombic 2-D shapes bounded by (111) faces (Figure 4). In addition, some crystals showed rounded corners corresponding to (110) crystal face (Figure 4). MamC-mediated nanoparticles expressed the (111) crystal face with rounded corners corresponding to nascent (110) and (311) crystal faces (Figure 4). In this case, crystals appeared elongated along [111] direction. Crystals obtained in the presence of Mms6 protein showed rhombic, rectangle and hexagon shapes bounded by (111) crystal face and rounded corners corresponding to (311), (110) and (400) crystal faces (Figure 5). These crystals were elongated along [111] direction. Nanoparticles obtained at 5 µg/mL of MamC and 10 µg/mL of Mms6 had shapes and corners that were more defined than those seen in the nanoparticles obtained when only one of the proteins was present. In particular, crystals from this experiment showed rhombic, rectangle and hexagon shapes bounded by (111) crystal faces and were elongated along [111] direction. The well-defined corners observed correspond to (110), (311) and (400) crystal faces (Figure 5).

### Example 6: Magnetic measurements

Magnetization measurements were carried out by using a Quantum Design Superconducting Quantum Interference Device (SQUID) 5T magnetic properties measurement system (MPMS). Under gentle argon flow, 1.6 mg of MNPs and 1.01 mg of BMNPs was placed in a double-walled polycarbonate capsule. Hysteresis cycles for each type of nanoparticles were determined at 5 K and 300 K.

Zero-field cooling (ZFC-W) and field cooling (FC-C) measurements were carried out using a superconducting quantum interference device (SQUID) 5 T magnetometer (Quantum Design MPMS XL, USA). Under gentle argon flow, a different amount of each specimen powder was placed in a double-walled polycarbonate capsule. The samples were immediately cooled in a zero applied field to 5 K to preserve randomized magnetization of the nanocrystals, after which a 500 Oe magnetic field was applied. To allow comparison among the differently synthesized nanoparticles, the M(T) curves were normalized by the amount of each sample analyzed and by the magnetization value at 300 K.

Nanoparticles synthesized in the inorganic control experiment exhibit the lowest blocking temperature (T_{B} ∼ 50 K) which is characteristic of small and poorly crystalline nanoparticles (Figure 6). Mms6-mediated nanoparticles show similar magnetization curves (Figure 6) while MamC-mediated nanoparticles exhibit a higher T_{B} (∼ 140 K), consistent with their larger size. The nanoparticles obtained at 5 µg/mL of MamC and 10 µg/mL of Mms6 show the highest T_{B} (T_{B} ∼ 300 K) with the slowest increase of magnetization, characteristic of particles with high crystallinity and a large magnetic moment per particle.

Both MNPs and BMNPs present remanent magnetization at 5 K in the absence of an external field, but not at 300 K (Figure 6C), which confirms that both particles are superparamagnetic and have a blocking temperature < 300 K. According to the data obtained, the saturation magnetization value (Ms) for BMNPs is 55 emu/g, while for MNPs it is 66 emu/g (Figure 6C). The difference in saturation magnetization among BMNPs and MNPs is not so high considering the dilution effect of the coating, so the reduction in the Ms value of the BMNPs could be caused by the incorporation of MamC. In fact, considering the percentage of MamC incorporated according to TGA data (BMNPs 9.4% and MNPs 4.5%; Figure 6B) the corrected values of Ms for BMNPs and MNPs should be respectively 55/(1-0.094) = 61 emu/g and 66/(1-0.045) = 69 emu/g, which indicates that they are identical within the experimental error range.

Moreover, the blocking temperatures (T_{B}) and the irreversibility temperatures (Tᵢᵣᵣ) of the biomimetic particles and MNP are also different. The lowest T_{B} (103 K) and Tᵢᵣᵣ (274 K) correspond to MNPs (Figure 6D), then to Mms6-BMNPs, and then MamC-BMNPs (Figure 6D), while the highest T_{B} (260 K) and Tᵢᵣᵣ (296 K) correspond to Mms6-MamC-BMNPs. The slowest magnetization and higher T_{B} values correspond to particles with a larger magnetic moment per particle. Furthermore, the lower differences between T_{B} and Tᵢᵣᵣ indicate lower polydispersity.

### Example 7: Specific surface area measurements

Powdered samples were analyzed to obtain nitrogen sorption isotherms at 77 K on a TriStar 3000 equipment (Micromeritics). About 50 mg of samples were degassed at 100°C for 4 h prior to analysis using a sample degas system (VacPrep 061, Micrometrics). The specific surface area (SSA) of the samples was determined using the BET method [27]. The SSA determined by BET is 97 ± 2 m²/g.

### Example 8: Electrophoretic mobility measurements and thermogravimetric analyses

Electrophoretic mobility was measured in inorganic magnetites (MNPs) and BMNPs. Stock suspensions of each type of the nanoparticles were prepared in 10 mL of oxygen-free NaClO₄ (10 mM). Aliquots of 200 µL of each of the aforementioned suspensions were inoculated in eleven tubes, each one containing oxygen-free NaClO₄ (10 mM), the final volume of each tube being 10 mL. The pH of each tube was adjusted by adding oxygen-free HCI (0.1 M) or oxygen-free NaOH (0.1 M) until achieving a pH ranging from 2 to 11 depending on the sample. Samples were sonicated for 2 minutes before the measurements. Nine replicas were performed per each measurement.

Thermogravimetric analyses (TGA) were run on □10 mg of solid, heating the sample in an alumina cell under N₂ atmosphere, at a rate of 20°C min-1 up to a final temperature of 950°C.

The plots of ζ-potential versus pH (Figure 6A) reveal significant differences among the values measured for MNPs and BMNPs. They are both are positively charged at low pH values and negatively charged at high pH values but the isoelectric point (iep) differs. While this iep is 7.0 for MNPs, this value is 4.4 for BMNPs. These findings suggest that MamC is strongly attached to (or maybe incorporated into) the crystals. This observation is confirmed by TGA analyses (Figure 6B). The total weight % (wt%) loss of BMNPs is 9.4, while for MNPs it is 4.5, indicating that BMNPs are composed of 95.1 wt% of magnetite and 4.9 wt% of MamC. Therefore, MamC seems to have an important role in controlling not only the nanoparticle size distribution but also their surface properties.

### Example 9: Magnetoliposomes

### (1) Biomimetic magnetoliposomes

Magnetite nanoparticles tend to aggregate because of their magnetic properties and it is necessary to apply an additional treatment to prevent such an aggregation before producing the magnetoliposomes. With this goal, biomimetic nanoparticles were incubated in 5 mL of 100 mM glutamate for 12 hours. The nanoparticle concentration was 4.5 mg/mL. After that, the particles were washed three times with water to remove the glutamate. After washing, the particles were concentrated by using a magnet and the supernatant was discarded. As said, this procedure was repeated three times. Then, particles were re-dispersed in 1.67 mL of water ([Magnetite nanoparticles]≈24 mg/mL). This suspension was filtered through a filter with a pore size of 0.22 µm.

Biomimetic magnetoliposomes were synthesized by the thin film hydration method. To obtain the thin lipid film layer, phosphatidylcholine(PC) was dissolved in 8 mL of chloroform ([PC]=1.25 mg/mL) forming a homogenous suspension. The solvent was evaporated by using a rotavapor (Büchi, Rotavapor-R) under a vacuum current at 400 rpm and 37°C. In addition, to eliminate any chloroform residue, the sample was under a vacuum current for 90 minutes. Then, the thin lipid film layer was hydrated and dispersed with the ferrofluid suspension ([PC]≈6 mg/mL). To ensure a correct dispersion, the mixture was shaken for 2 hours at 180-200 rpm. After that, the magnetoliposome suspension was kept at 4°C for 24 hours. Finally, unilamellar magnetoliposomes were obtained by using the extrusion method. Namely, the magnetoliposome solution was passed 5 times through 200 nm and 100 nm polycarbonate membrane filters (Whatman), respectively, using an extruder (Avanti Polar Lipids) at 45°C.

### (2) Inorganic magnetoliposomes

Inorganic nanoparticles, like biomimetic nanoparticles, tend to aggregate. However, the treatment applied to disaggregate them is different from that followed for biomimetic nanoparticles because of the different surface properties of both particles. Thus, the inorganic nanoparticles were incubated in 5 mL of 2 M citrate. The rest of the protocol to obtain inorganic magnetoliposomes was identical to that followed to obtain biomimetic magnetoliposomes.

### Example 10: Functionalization of particles with doxorubicin and DO-24

The nanoparticles tested were those obtained using 10 µg/mL of MamC protein. The resulting precipitates were concentrated in tubes with a magnet and the supernatant was discarded. Then the precipitates were washed sequentially with oxygen-free Milli-Q water three times, 0.5% SDS solution and oxygen-free water again. Finally precipitates were resuspended in HEPES buffered saline solution (0.01 M HEPES, pH 7.2, 0.15 M NaCI) and sterilized by autoclaving them at 121°C for 21 min.

Magnetite nanoparticles were functionalized with DOXO and with the purified DO-24 monoclonal antibody (mAb), which recognizes the ectodomain of the human Met/HGF receptor, which is considered a tumor marker, being overexpressed in many cancers, as already described with minor modifications (lafisco *et al.,* 2010; lafisco *et al.,* 2013; Oltolina *et al.*, 2015).Briefly, couplings were performed mixing 2 mg of magnetite nanoparticles with 1 mg/mL of DOXO dissolved in water or mAb dissolved in HEPES buffered saline solution (binary magnetite nanoparticles) or mAb followed by DOXO (ternary magnetite nanoparticles) inside hermetically closed bottles to avoid magnetite oxidation. Adsorption kinetics experiments were carried out at 25°C with shaking (200 rpm) for different periods of time up to 24 h. At the end of each incubation period, mixtures were washed three times to separate the particles from the supernatants using a magnet. The amounts of adsorbed DOXO and mAb were assessed by UV-Vis spectroscopy (λ = 490 and 280 nm, respectively), calculating the differences between the concentrations of the molecules in the solution before and after the adsorption on magnetite nanoparticles (the mentioned supernatant). The functionalized nanoparticles were resuspended in HEPES buffered saline solution and kept at 4°C until use.

In the case of magnetite nanoparticles conjugated with both motifs (DOXO and mAb) (the so-called ternary nanoparticles), the same protocols were sequentially applied, each one followed by extensive washings. DO-24 mAb was coupled first and DOXO in the second step, based on previous experiments (lafisco *et al.,* 2013).

### Example 11: Cytocompatibility and cytotoxicity of the binary and ternary nanoparticles

**Cell cultures:** the GTL-16 cell line, which was derived from a poorly differentiated human gastric carcinoma which express Met, and the Huh-7 cell line, which was derived from a well differentiated hepatocellular carcinoma which is negative for Met, were maintained in Dulbecco's modified Eagle's medium (DMEM), supplemented with 10% fetal calf serum (FCS), 50 U/ml penicillin, and 50 µg/mL streptomycin. Cells were transplanted when they reached 80-90% confluence.

Cells (approximately 12 × 10³ GTL-16/well or 6 × 10³ Huh-7/well) were incubated in 96-well plates for 24 h; then different concentrations of the different magnetite nanoparticles, either naïve or functionalized were added in 100 µL to each microwell. After 3 days of incubation, cell viability was evaluated by the 3-(4, 5-dimethylthiazol-2-yl)-2,5-diphenyltetrazolium) bromide (MTT) colorimetric assay, as described in Oltolina *et al.,* 2015. [Briefly, 20 µL of MTT solution (5 mg mL⁻¹ in PBS solution) was added to each well. The plate was incubated at 37°C for 2 h and then supernatants were carefully aspirated. Afterwards, 125 µL of 0.2 N HCI in isopropanol was added to dissolve the formazan crystals formed. An aliquot of 100 µL was then removed carefully from each well and the optical density was measured in a multiwell reader (2030 Multilabel Reader Victor TM X4, PerkinElmer) at 570 nm. Viability of parallel cultures of untreated cells was taken as 100% viability and values obtained from cells undergoing the different treatments were referred to this value. Experiments were performed 3-5 times using 3 replicates for each sample. In some experiments predefined or equimolar amounts of soluble DOXO were used.

For experiments with XCELLIGENCE® instrument cells, approximately 12 × 10³ GTL-16/well or 6 × 10³ HuH-7/well, were seeded on appropriate multi-well plates for 24 h. From this time the impedance was monitored (time 0 of the experiment), and the different functionalized magnetite nanoparticles were added in 100 µL to each microwell. Equimolar amounts of DOXO, either soluble or adsorbed on nanoparticles, were also used.

When the binary and ternary magnetite nanoparticles, both of them carrying DOXO, were incubated for 3 days on the cells, they behaved similarly, i.e., exerted the same level of toxicity, with respect to either the untreated reference samples or the samples treated with soluble DOXO, except in the case when 10 µg/ml DOXO were used on GTL-16 cells (Figure 9). In this case ternary mAb-functionalized magnetite nanoparticles were significantly more toxic than binary magnetite nanoparticles carrying only DOXO; no such difference was observed in the case of Huh7, which do not express the receptor targeted by the mAb. In all cases both types of magnetite nanoparticles displayed toxicity in a dose-dependent manner and toxicity exerted by DOXO from magnetite nanoparticles was lower than that exerted by soluble DOXO, in accordance with that already reported by the research team and others for other types of functionalized nanoparticles. Thus, specificity of toxicity was restricted to a narrow dose of ternary magnetite nanoparticles. Since only end point data are obtained in MTT experiments, experiments monitoring in real-time the cytotoxic response kinetics of the magnetite nanoparticles using the XCELLIGENCE apparatus were then performed. In this case in experiments performed with GTL-16 cells at doses of 100 µg/ml DOXO, ternary mAb-functionalized magnetite nanoparticles were significantly more toxic than binary magnetite nanoparticles carrying only DOXO and as efficient as soluble DOXO up to 48 h. On the third day the three treatments with DOXO, either soluble or coupled to both types of magnetite nanoparticles induced the same level of toxicity, in line with the data of the MTT assay. When the same experiments were performed on Huh7 cells, no significant differences were observed between the effects exerted by the two types of functionalized magnetite nanoparticles, which behaved similarly and were only slightly less toxic then soluble DOXO. All together these data indicate that mAb functionalization provides specificity to the magnetite nanoparticles, endowing them with the ability to target cells expressing the complementary antigen.

### Example 12: In vivo biocompatibility and nanoparticle distribution

Female BALB/c mice were injected in the tail vein with magnetite nanoparticles (10 µg nanoparticles/g mouse weight) diluted in a final volume of 100 µl of sterile PBS. Animals were monitored every second day up to 1 month. Mice were subdivided into 5 groups, differing by the time point of euthanization (from 1 h to 2 months). For each group composed of 3 mice, one control untreated mouse was also used. Their organs were collected, fixed, embedded in paraffin, and processed for histological analysis. Serial sections were stained with Prussian blue and hematoxylin-eosin (Sigma Aldrich) and subjected to histological evaluation by an independent pathologist not informed of the sample identity. All procedures were carried out in accordance with the European Community Directive for Care and Italian Laws on animal experimentation (Law by Decree 116/92).

All mice injected with magnetite nanoparticles(10 µg/g mouse weight) were found to be alive and in good shape for at least 60 days. Sections of brain, heart, lung, spleen, liver and kidney prepared from animals 1, 4 h, 1, 7, 60 days after the injection do not show any morphological alterations compared to the ones from control mice (Figure 11). Few magnetite nanoparticles were detected, mainly as aggregates in the lungs. Moreover, in the case of the spleen, which in control untreated animals was already positive for Prussian Blue staining, such a staining was undetectable 4 h and 1 day after magnetite nanoparticle injection, but it was detectable at least 1 week after, if not before. All together these data confirm the minimal/low toxicity of magnetite nanoparticles up to10 µg/g mouse weight.

### 4T1 Cell cultures preparation

4T1 cells (ATCC® CRL-2539™) are a murine breast carcinoma cell line derived from BALB/c mice, which presented a tumor growth and metastatic spread very closely mimicking the stage IV of human breast cancer. These cells were maintained in Dulbecco's Modified Eagle Medium (DMEM), supplemented with 10% fetal calf serum (FCS), 50 U/ml penicillin, and 50 µg streptomycin. Cells were transplanted twice a week, when they were at 90-95% confluence.

### Example 13. In vitro cytocompatibility of BMNPs in the absence/presence of magnetic fields

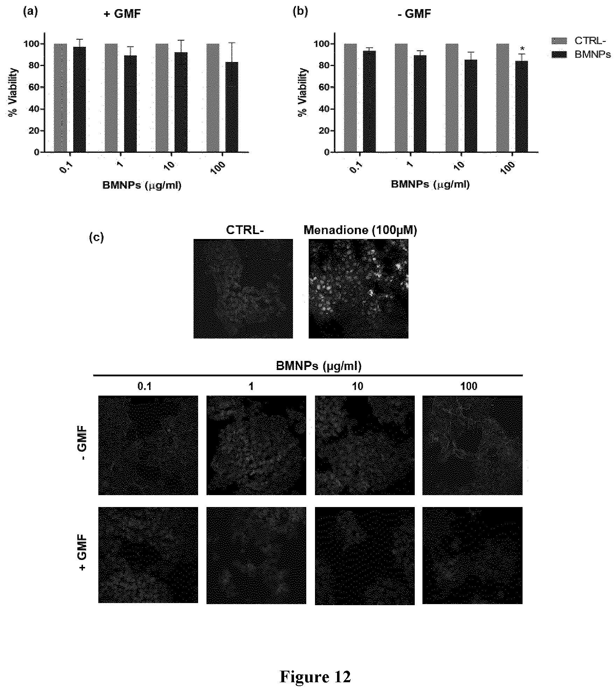

The BMNPs did not display significant toxicity (viability always over 80%) in an MTT assay up to 100 µg/mL concentrations in 4T1 cells (Figure 12a). Moreover, the same level of cell viability was observed when an external gradient magnetic field was applied using a neodymium magnet (1.8 Kg pull) for 72 h (Figure 12b), confirming its safe use.

The level of ROS was also measured in the cells treated as above, since the production of these molecules is a sign of the oxidative stress for the cells. No ROS release was detectable in both conditions either with or without exposure to an external magnetic field. While in the positive control treated with Menadione (100 µM), which induces oxidative stress, a virtual green color (CellROX® Green Reagent) due to the ROS was clearly visible under confocal microscopy (Figure 12c). Therefore, it can be concluded that the BMNPs are cytocompatible (Figure 12) and do not induce oxidative stress in living cells.

The viability of cells subjected to an alternating magnetic field of 130 kHz and 16 kAm-1 was also analyzed. In this case 4T1 cells (10⁶) were resuspended in 0.15 mL tubes together with BMNPs at different concentrations and placed inside the coil of the instrument for 20 min. An aliquot containing 10,000 cells was then recovered and plated for an MTT assay which was read the following day. Cells were fully viable when incubated with 100 µg of BMNPs, no cytotoxicity was observed, both in the presence or absence of the alternating magnetic field, because that BMNP concentration was not enough to generate heat by rotation (Figure 13). On the other hand, by increasing the amount of BMNPs in the presence of the alternating magnetic field, cell viability was significantly reduced in a dose-dependent manner, reaching 8.4% at 500 µg. In any case this high amount of BMNPs required to display cytotoxicity by hyperthermia induced some toxicity by itself, although the latter was significantly lower (Figure 13).

### Example 14. Cellular interaction of BMNPs and DOXO-BMNPs in the absence/presence of a gradient magnetic field

The interaction of BMNPs with cells in the presence/absence of a gradient magnetic field is shown in Figure 14. Cells plated on coverslips were incubated for different periods of time with BMNPs in the presence or absence of a magnetic field, fixed, washed and stained with Prussian blue (Figure 14a). In the treatment without the magnetic field, BMNPs are detectable only and at a very low level after 1 min incubation, while in the case of the application of the magnetic field they are clearly visible already after 5 seconds, the first time analyzed. In both cases more BMNPs are detectable with an increasing time of incubation, but there was always a significant difference between samples that were treated or not treated with the magnetic plate, and indeed at 5 min without the application of the magnetic field there were fewer BMNPs than in samples treated for 5 second with the magnet. Iron quantification experiments were carried out using potassium thiocyanide on samples treated as before (Figure 14b). In the absence of the magnetic plate, a low amount of iron was detected until 1 min incubation, and then it increased up to 46 µg/mL at 5 min. When cells were treated with the magnetic plate, a significant amount of iron was associated with the cells from the very start (36.2 µg/mL at 5 second) and iron continued to be accumulated in a time-dependent manner, until stabilization (60 µg/mL) after 150 seconds.

The interaction of BMNPs with cells was also analyzed by TEM at different times and energy dispersive X-ray (EDX) (Figure 15). In accordance with the 30-second optical microscopy data, few BMNPs are around the cell surface when cells are not subjected to the magnetic field. Otherwise, some BMNPs appear to interact with the cell membrane and even appear to be internalized if cells underwent treatment with the magnetic field (Figure 15a). In contrast, Prijic *et al.* (2010) did not observe statistically significant differences up to 30 seconds. Although superparamagnetic, the NPs used in that study have sizes of 8-9 nm, which have a lower magnetic response than single magnetic domain NPs, coated with a 2-nm-thick layer of silica that also affects the magnetic behavior.

At 24 h the concentration of internalized particles was comparable between samples in the absence and presence of the magnetic field (Figure 15a). These results are in accordance with Prussian blue analysis and iron quantification, in which a higher cell-particle interaction is observed at short periods of time, up to 2.5 min. All together these data show that BMNPs are highly responsive to a gradient magnetic field *in vitro,* which allows for a faster cellular interaction (Figure 14) and uptake (Figure 15).

### Example 15. In vitro cytotoxicity of DOXO-BMNPs in the absence/presence of magnetic fields

Cells were incubated with different concentrations of DOXO-BMNPs for 72 h in the presence or absence of the magnetic plate and cytotoxicity was measured in an MTT assay. At all the concentrations tested, no differences in cytotoxicity in the presence or in the absence of the magnetic field were observed (Figures 16a, 16b). This is reasonable since the effect of the magnetic field on the interaction with cells was only observed in short periods of time, as observed after Prussian blue staining. Therefore, to evaluate potential differences between the cytotoxicity in the samples treated and not with the magnetic field, shorter time points (5, 30, 60, 150, and 300 seconds) and one BMNP concentration (100 µg/mL) were used (Figures 16c, 16d). After magnetic treatment the medium and the BMNPs were removed and incubation with fresh media was continued for 72 h for an MTT assay. In the presence of the magnetic field at the time points of 5 and 30 seconds, the cytotoxicity exerted by the DOXO-BMNPs is exactly the same as the one induced by soluble DOXO, while in the absence of the magnetic field DOXO-BMNPs exert a lower toxicity compared to soluble DOXO at the first two times (Figures 16c, 16d). Thus treatment with the magnetic field concentrated or even internalized the BMNPs at close contact with the cells, as already verified by Prussian blue (Figure 14) and TEM (Figure 15) analyses, so that DOXO was readily available for toxicity.

When the internalization of soluble DOXO and of DOXO coupled on BMNPs was analyzed by confocal microscopy, in all the samples tested, the DOXO signal increased over the time (Figure 17). However, interestingly, the internalization of DOXO in the cells nuclei was already detected at 0.5 and 5 minutes in the DOXO-BMNPs treated with the magnetic field, while it was detected only after 5 min in the case of soluble DOXO and at neither times when DOXO-BMNPs were incubated in the absence of the magnetic plate (Figure 17). In this case the DOXO signal of the functionalized BMNPs was detectable only after 30 minutes of incubation and its intensity was lower (Figure 17). Therefore, magnetic field treatment accelerates uptakes of DOXO from the BMNPs and nuclear accumulation thereof. These results are also directly related to a faster cytotoxic activity of DOXO-BMNPs under the influence of a gradient magnetic field for short periods of time, analyzed by MTT assay (Figure 16c,d).

### Example 16. Effects of gradient and alternating magnetic fields in vivo on tumors

In the first *in vivo* experiment the gradient continuous magnetic field was applied or not for 1h to 4T1 cell-induced tumors, after the i.v. injection of the different NPs or of soluble DOXO (2 mg/kg mouse). The treatments were repeated 5 times, every 3 days and every time tumor sizes were evaluated and compared to control animals receiving only PBS. Up to day 6 (measured just before the second injection) no differences between the groups receiving the different treatments were observed (Figure 18a). From day 9 to day 12, the apposition of a direct current magnetic field coin on the tumors of animals receiving either naked or DOXO-coupled BMNPs inhibited their growth at a higher level when compared to mice not subjected to magnetic field treatment. At the end of the experiment (day 15) tumor volumes were significantly reduced only in the groups of mice receiving DOXO. In particular, the highest inhibition was observed in mice receiving the combined treatment of DOXO-BMNPs and apposition of the magnetic coin, in comparison to animals receiving only DOXO-BMNPs or soluble DOXO (Figure 18a). On the last day of the experiment the mice were euthanized, their tumors excised, fixed and their histological sections were stained with Prussian blue to analyze and quantify their iron content. As expected, sections from tumors in animals which underwent magnetic field treatment displayed a higher content of the blue pigment revealing iron, which was similar in the two cases of naked or DOXO-coupled BMNPs (Figures 18b, 18c).
On the other hand, these results suggest that the DOXO adsorbed onto the BMNPS did not interfere with the magnetic field. This means that the magnetic field can direct the BMNPs/DOXO-BMNPs to a specific organ/tumor for drug delivery or hyperthermia treatment (once it has arrived at the tumor site). This targeted treatment potentially reduces the side effect of the drug on healthy cells in the rest of the body, thus favoring the accumulation of nanoparticles in the tumor site and of the coupled DOXO, which could exert its toxic effect. In this context, a significant amount of work has been done for the purpose of achieving a high concentration of drug in the affected area with a rapid response time and minimal side effects through the application of the magnetic field.

When an alternating magnetic field was applied *in vivo,* hyperthermia production was observed only in the mice injected with the nanoparticles (Figure 19a,b). Tumor temperature rose rapidly and was maintained at 42-45°C for 20 minutes in the presence of the alternating magnetic field. This increase in temperature had an effect on reducing tumor size when measured 3 days post injection (Figures 19c, 19d). However, when hyperthermia is combined with the cytotoxicity of DOXO, i.e. DOXO-BMNPs + alternating magnetic field, a synergistic effect was observed, reducing the tumor at higher levels compared to BMNPs + alternating magnetic field, and at the same levels as soluble DOXO (Figure 19c). Moreover, 5 days post-injection, the only group with a statistically significant reduction in tumor weight is the group that combined hyperthermia + DOXO (Figure 19d).

These *in vivo* data represent the basis for the potential safety and efficient application of BMNPs as dual antitumor therapy, which combines magnetic drug targeting with magnetic hyperthermia treatment to increase efficiency.

### More details about the described examples:

### Cellular interaction of BMNPs and DOXO-BMNPs in the absence/presence of a gradient magnetic field

### Prussian blue staining

Cells (approximately 20 × 10³ 4T1/well) were seeded on glass coverslips in 24-well plates and, after 24 h, 100 µg/mL BMNPs suspensions were added. After incubation at 37°C for short (5 and 30 seconds) and longer (1, 2.5, and 5 minutes) periods of time in the absence (- GMF) and in the presence (+ GMF) of a gradient magnetic field, coverslips were washed with fresh phosphate buffered saline (PBS) pH 7.2 and fixed with paraformaldehyde (2 wt% in PBS). Then Prussian blue solution (1:1 of 2% potassium ferrocyanide in H₂O and 2% HCI both in H₂O) was added to the coverslips. In that way any ferric ion (+3) present in the samples combines with the ferrocyanide and results in the formation of bright blue pigments called Prussian blue or ferric ferrocyanide. After two other washes with fresh PBS, Nuclear Fast Red was added for staining cell nuclei. Finally, coverslips were washed with H₂O and mounted on slides using one drop of Eukitt quick-hardening mounting medium for each sample. The interaction of the stained BMNPs with cells was analyzed by optical microscopy at 100X.

### Iron quantification by potassium thiocyanide

Cells (approximately 22 × 10⁴ 4T1/well) were seeded in 6-well plates and, after 24 h of incubation at 37°C and 5% CO₂, 100 µg/mL BMNPs suspensions in DMEM medium were added. After their incubation for 5, 30, 60, 150, and 300 seconds in the presence and absence of a gradient magnetic field, the supernatant was removed, cells were washed with fresh PBS, trypsinized, transferred to 0.5 mL tubes and centrifuged at 1000 for 5 min. Then, the cell pellets formed were dissolved in 37% HCI, mixed with 10% H₂O₂, and incubated for 20 min at room temperature. After the incubation time, the samples were colored with 1 mL of 1% potassium thiocyanide in MilliQ water, and their absorbance was measured at 490 nm. The concentration of ferric ions, i.e., BMNPs, was calculated referencing the absorbance obtained to a standard curve performed with BMNPs alone. The endogenous iron of the cells was subtracted from the treated samples normalizing by the untreated control cells.

### Confocal analysis

Cells (approximately 20× 10³ 4T1/well) were seeded on glass coverslips in 24-well plates and, after 24 h, soluble DOXO (as a positive control) or DOXO-BMNP suspensions were added. After incubation at 37°C for different periods of time (30 seconds, 5 and 30 minutes) in the absence (- GMF) and in the presence (+ GMF) of a gradient magnetic field, coverslips were washed with fresh PBS pH 7.2 and fixed with paraformaldehyde (2% wt in PBS). To minimize unspecific interactions and permeabilize cells, coverslips were washed with Tris-Buffered Saline (TBS) containing 5% Bovine Serum Albumin (BSA), 0.1% Triton X-100 and 5% got serum and then stained. In particular cytoskeletal actin microfilaments were stained with FITC-phalloidin (Sigma-Aldrich, excitation at 488 nm; emission at 500-535 nm) and nuclei were stained with TO-PRO-3 (1/70, Life Technologies; excitation at 633 nm; emission at 650-750 nm). DOXO was detected after excitation at 476 nm and emission at 575-630 nm. Fluorescence was detected using a Leica TCS SP2 AOBS Spectral Confocal Scanner microscope. Images were taken at 400X magnification. ImageJ software was used for analysis.

### Cellular internalization of BMNPs in the absence/presence of a gradient magnetic field

Cells (approximately 10 × 10⁵ 4T1/well) were incubated at 37°C and 5% CO₂ for 24 h. Afterwards, 100 µg/mL of BMNPs were added and incubated in the absence and presence of a gradient magnetic field for 30 seconds, 1 and 24 h. After these treatments, cells were washed three times with PBS prior to fixation with 2.5% glutaraldehyde and 2% paraformaldehyde in PBS for 1 h. Then samples were washed again three times with sodium cacodylate buffer and embedded in epon. Ultrathin sections (50-70 nm) were cut using a Reichert Ultracut S microtome (Leica Microsystems GmbH, Wetzlar, Germany), mounted on copper grids, and stained with lead citrate and uranyl acetate for transmission electron microscopy (TEM) analysis. In addition, microanalysis by energy dispersive X-ray (EDX) spectroscopy was performed to confirm BMNP imagining by iron detection.

### MTT assay in the absence/presence of a gradient or an alternating magnetic field

Cells (approximately 5 x 10³ 4T1/well) were incubated in 96-well plates for 24 h. Then different concentrations (0.1, 1, 10, and 100 µg/mL) of soluble DOXO, BMNPs, and DOXO-MNPs were added to plated cells in 100 µL. Equimolar amounts of DOXO, either soluble or loaded on nanoparticles, as well as of BMNPs, were used. These samples were incubated at 37°C and 5% CO₂ in the absence or presence of a gradient magnetic field, using a magnetic plate below the 96-well plates, for 72 h. In another set of experiments the cells were incubated with 100 µg/mL BMNPs for shorter time points (5, 30, 60, 150, and 300 seconds) in the presence and absence of the gradient magnetic field.

In the case of the alternating magnetic field treatment, approximately 95 x 10⁴ 4T1 cells were placed in a 0.5 mL tube. Then suspensions of 100, 300, and 500 µg of BMNPs in DMEM medium were added and exposed or not to an alternating magnetic field (130 kHz and 16 kAm⁻¹) for 20 minutes. After this treatment the cells were counted using trypan blue, seeded in 96-well plates (approximately 10 x 10³ 4T1/well) and incubated at 37°C and 5% CO₂ for 24 h.

At the end of the incubation time of the different sets of experiments, cell viability was evaluated by the MTT colorimetric assay. Briefly, 20 µL of MTT solution (5 mg mL⁻¹ in PBS solution) was added to each well. The plate was then incubated at 37°C for 2 h and then supernatants were carefully aspirated. Afterwards, 125 µL of 0.2 N HCI in isopropanol was added to dissolve the formazan crystals formed. 100 µL were then removed carefully and the optical density was measured in a multiwell reader (2030 Multilabel Reader Victor TM X4, PerkinElmer) at 570 nm. Viability of parallel cultures of untreated cells (CTRL-) was taken as 100% viability and values obtained from cells undergoing the different treatments were referred to this value. Experiments were performed 3 times using 3 replicates for each sample.

### Detection of reactive oxygen species (ROS) production

To measure the potential oxidative stress in living cells, as a consequence of the presence of the BMNPs, CellROX® Green Reagent (ThermoFisher) was used following the protocol recommended by the manufacturer. Briefly, cells (approximately 20 x 10³ 4T1/well) were seeded on glass coverslips in 24-well plates. After their exposure to different concentrations (0.1, 1, 10, 100 µg/mL), in the presence and absence of a gradient magnetic field, of BMNPs for 4 hours, the cells were washed with PBS and CellROX® Green Reagent was added to a final concentration of 5 µM in 300 µl of DMEM medium without serum. Then, the plate was incubated in the dark at 37°C for 30 minutes. Menadione (100 µM) was used as a positive control. After the incubation time, the coverslips were washed with PBS pH 7.2, fixed with 4% paraformaldehyde in PBS, washed again and permeabilized with 0.1% Triton-X100 for 10 minutes. Finally, the coverslips were stained and mounted on specimen slides (Biosigma). The cytoskeletal actin was stained with TRITC-phalloidine (1/200, Sigma-Aldrich, excitation at 543 nm; emission at 560-620 nm) and the nuclei with TO-PRO-3 (1/50, Life Technologies, excitation at 642 nm, emission at 650-750 nm). The CellROX® Green Reagent is only fluorescent in the oxidized state (as a consequence of ROS production). Therefore, the emission of green fluorescence (at 485/520 nm) is stable and is produced after the DNA binding, and therefore its signal is mainly located in the nucleus and in the mitochondria. Fluorescence was detected using a Spectral Confocal Leica TCS SP2 AOBS microscope. The images were taken at 400X magnification. ImageJ software was used for the analysis.

### In vivo magnetic targeting and antitumor activity

Female BALB/c mice were inoculated with 10⁵ 4T1 cells into a mammary gland fat pad. When the tumors became palpable (10 days after cell inoculation), mice were divided into 6 different groups with comparable tumor volumes among the groups. The 6 groups were intravenously injected and treated as follows: i) PBS (control), ii-iii) DOXO-BMNPs +/- gradient magnetic field, iv-v) BMNPs +/- gradient magnetic field, and vi) soluble DOXO. Mice were injected 5 times with a dose of 2 mg/kg DOXO which was soluble or adsorbed onto the BMNPs or comparable doses of unfunctionalized BMNPs, each time 3-4 days apart and, in case of magnetic treatment, after each injection they received a magnetic field treatment for 1 h. The magnetic field was applied by attaching a 10 mm in diameter x 3 mm thick N42 neodymium magnet (1.8 kg pull, Magnet Expert Ltd) with 3MTM VetbondTM tissue adhesive on the tumor site and keeping it attached for 1 hour after the injection. This neodymium magnet, with a magnetic anisotropy normal to the plane and a saturation magnetization of 800 emu/cc, could generate a direct current (d.c.) magnetic field of the order of 100 Oe a few millimeters from the surface. Therefore, the effect of the magnet is equivalent to the application of a local 100-Oe external d.c. magnetic field immediately after the administration of the nanoparticles. Throughout the study, body weight and tumor volumes (measured with caliper) were recorded every 3-4 days. Three days after the last injections (day 18), mice were euthanized, their weights, as well as, tumor weights were recorded and, then, tumors, hearts, livers, spleens, brains, lungs, and kidneys were collected for histology. Histological sections of the tumors were prepared for hematoxylin-eosin and Prussian blue staining to analyze particle biodistribution.

### In vivo hyperthermia and antitumor activity

36 female BALB/c mice were inoculated with 10⁵ 4T1 cells into a mammary gland fat pad. After ∼15 days after cell inoculation, when the tumor dimensions were ∼100 mm³, mice were divided into 6 different groups with comparable tumor volumes among the groups. The 6 groups were intratumor injected and treated as follows: i) PBS (control), ii-iii) DOXO-BMNPs +/- alternating magnetic field, iv-v) BMNPs +/- alternating magnetic field, and vi) soluble DOXO. Mice were injected only once at the beginning of treatment (day 0) with a dose of 3 mg BMNPs/mouse, equivalent to 80 µg DOXO for soluble DOXO and DOXO-BMNP groups. After each injection, some groups were exposed to an alternating magnetic field (130 kHz and 16 kAm⁻¹) for 20 minutes immediately after the administration of the nanoparticles. Throughout the study, tumor volumes were measured with a caliper every two days. Finally, five days post-treatment, mice were euthanized and their tumor weights recorded.

### Statistical analysis

One-way ANOVA statistical analyses were performed with Bonferroni's or Dunnett's post-test using GraphPad Prism version 4.03 (San Diego, CA). Statistical differences between the treatments were considered significant when p values were p<0.05 (*), p<0.01 (**), p<0.001 (***).

### References

Alphandéry, E., Faure, S., Seksek, O., Guyot, F., Chebbi, I., 2011. Chains of magnetosomes extracted from AMB-1 magnetotactic bacteria for application in alternative magnetic field cancer therapy. ACS Nano 5, 6279-6296.
Amemiya, Y., Arakaki, A., Staniland, S., Matsunaga, T., 2007. Controlled formation of magnetite crystal by partial oxidation of ferrous hydroxide in the presence of recombinant magnetotactic bacterial protein Mms6. Biomaterials 28, 5381- 5389.
Arakaki, A., Masuda, F., Amemiya, Y., Tanaka, T., Matsunaga, T., 2010. Control of the morphology and size of magnetite particles with peptides mimicking the Mms6 protein from magnetotactic bacteria. J.Colloid Interface Sci. 343, 65-70.
Arakaki, A., Yamagishi, A., Fukuyo, A., Tanaka, M., Matsunaga, T., 2014. Coordinated functions of Mms proteins define the surface structure of cubo-octahedral magnetite crystals in magnetotactic bacteria. Mol. Microbiol. 93, 554-567.
Arató, B., Szányi, Z., Flies, C., Schüler, D., Frankel, R.B., Buseck, P.R., Pósfai, M., 2005. Crystal-size and shape distributions of magnetite from uncultured magnetotactic bacteria as a potential biomarker. Am. Mineral. 90, 1233-1241.
Bazylinski, D.A., Frankel, R.B., 2004. Magnetosome formation in prokaryotes. Nat. Rev. Microbiol. 2 (3), 217-230.
Brigger I, Dubernet C, Couvreur P. Nanoparticles in cancer therapy and diagnosis. Adv Drug Deliv Rev. 2002;54:631-51. Review.
De Jong WH, Borm PJA Drug delivery and nanoparticles: Applications and hazards Int J Nanomedicine. 2008;3:133-149.
Bird, S.M., Rawlings, A.E., Galloway, J.M., Staniland, S.S., 2016a. Using a biomimetic membrane surface experiment to investigate the activity of the magnetite biomineralisation protein Mms6. RSC Adv. 6, 7356.
Galloway, J.M., Bramble, J.P., Rawlings, A.E., Burnell, G., Evans, S.D., Staniland, S.S., 2012a. Nanomagnetic arrays formed with the biomineralization protein Mms6. J. Nano Res. 2012 (17), 127-146.
Galloway, J.M., Bramble, J.P., Rawlings, A.E., Burnell, G., Evans, S.D., Staniland, S.S., 2012b. Biotemplated magnetic nanoparticle arrays. Small 8, 204-208.
lafisco, M., Varoni, E., Di Foggia, M., Pietronave, S., Fini, M., Roveri, N., Rimondini, L., Prat, M., 2012. Conjugation of hydroxyapatite nanocrystals with human immunoglobulin G for nanomedical applications. Colloids Surf B Biointerfaces 90, 1-7.
lafisco, M., Delgado-Lopez, J.M., Varoni, E.M., Tampieri, A., Rimondini, L., Gomez-Morales, J., Prat, M. 2013. Cell surface receptor targeted biomimetic apatite nanocrystals for cancer therapy. Small 25, 9 (22), 3834-3844.
Jimenez-Lopez, C., Rodriguez-Navarro, C., Rodriguez-Navarro, A., Perez-Gonzalez, T., Bazylinski, D. A., Lauer, H. V., Romanek, Jr. C. S. 2012. Signatures in magnetites formed by (Ca,Mg,Fe)CO3 thermal decomposition: Terrestrial and extraterrestrial implications. Geochimica et Cosmochimica Acta 87, 69-80.
Kolinko, I., et al. D., 2014. Biosynthesis of magnetic nanostructures in a foreign organism by transfer of bacterial magnetosome gene clusters. Nat. Nanotechnol. 9, 193-197.
Langmuir, I. The adsorption of gases on plane surfaces of glass, mica and platinum. J. Am. Chem. Soc 40, 1361-1403 (1918).
Lisy, M.R., et al., 2007. Fluorescent bacterial magnetic nanoparticles as bimodal contrast agents. Invest. Radiol. 42 (4), 235-241.
Martin Ramos, J.D. XPowder, a software package for powder X-ray diffraction analysis. Legal Deposit GR 1001/04 (2004).
Maruyama, K., Takeyama, H., Nemoto, E., Tanaka, T., Yoda, K., Matsunaga, T., 2004. Single nucleotide polymorphism detection in aldehyde dehydrogenase 2 (ALDH2) gene using bacterial magnetic particles based on dissociation curve analysis. Biotechnol. Bioeng. 87, 687-694.
Maruyama, K., Takeyama, H., Mori, T., Ohshima, K., Ogura, S., Mochizuki, T., Matsunaga, T., 2007. Detection of epidermal growth factor receptor (EGFR) mutations in non-small cell lung cancer (NSCLC) using a fully automated system with a nano-scale engineered biomagnetite. Biosens. Bioelectron. 22, 2282-2288.
Matsunaga, T., Suzuki, T., Tanaka, M., Arakaki, A., 2007. Molecular analysis of magnetotactic bacteria and development of functional bacterial magnetic particles for nano-biotechnology.Trends. Biotechnol. 25, 182-188.
Oltolina, F., Gregoletto, L., Colangelo, D., Gomez-Morales, J., Delgado-Lopez, J. M., Prat, M. 2015. Monoclonal Antibody-Targeted Fluorescein-5-isothiocyanate-Labeled Biomimetic Nanoapatites: A Promising Fluorescent Probe for Imaging Applications. Langmuir, 31 (5), 1766-1775.
Ota, H., Lim, T.K., Tanaka, T., Yoshino, T., Harada, M., Matsunaga, T., 2006. Automated DNA extraction from genetically modified maize using aminosilane-modified bacterial magnetic particles. J. Biotechnol. 125, 361-368.
Perez-Gonzalez, T.C., Jimenez-Lopez, C., Neal, A.L., Rull-Perez, F., Rodriguez-Navarro, A., Fernandez-Vivas, A., lañez-Pareja, E., 2010. Magnetite biomineralization induced by Shewanellaoneidensis. Geochim. Cosmochim. Acta 74 (3), 967-979.
Prozorov, T., Mallapragada, S.K., Narasimhan, B., Wang, L., Palo, P., Nilsen-Hamilton, M., Williams, T.J., Bazylinski, D.A., Prozorov, R., Canfield, P.C., 2007. Protein-mediated synthesis of uniform superparamagnetic magnetite nanocrystals. Adv. Funct. Mater. 17, 951-957.
Prozorov, T., Bazylinski, D.A., Mallapragada, S.K., Prozorov, R., 2013. Novel magnetic nanomaterials inspired by magnetotactic bacteria: topical review. Mater.Sci. Eng. R 74, 133-172.
Rawlings, A.E., Bramble, J.P., Walker, R., Bain, J., Galloway, J.M., Staniland, S.S., 2014. Self-assembled MmsFproteinosomes control magnetite nanoparticle formation in vitro. Proc. Natl. Acad. Sci. U.S.A. 111, 16094-16099.
Rill, C., Kolar, Z. I., Kickelbick, G., Wolterbeek, H. T. & Peters, J. A. Kinetics and thermodynamics of adsorption on hydroxyapatite of the [160Tb]terbium complexes of the bone-targeting ligands DOTP and BPPED. Langmuir 25, 2294-301 (2009).
Schwertmann, U., Cornell, R.M., 2000. Iron Oxides in the Laboratory: Preparation and Characterization, 2nd ed., vol. 188. Wiley-VCH, Weinheim, Germany.
Singh R, Lillard JW Jr. Nanoparticle-based targeted drug delivery. Exp Mol Pathol. 2009;86:215-23. Review.
Sun, J.B., Duan, J.H., Dai, S.L., Ren, J., Guo, L., Jiang, W., Li, Y., 2008. Preparation and antitumor efficiency evaluation of doxorubicin-loaded bacterial magnetosomes: magnetic nanoparticles as drug carriers isolated from Magnetospirillum gryphiswaldense. Biotechnol. Bioeng. 101 (6), 1313-1320.
Thomas-Keprta, K.L., Bazylinski, D.A., Kirschvink, J.L., Clemett, S.J., McKay, D.S., Wentworth, S.J., Vali, H., Gibson Jr., E.K., Romanek, C.S., 2000. Elongated prismatic magnetite crystals in ALH84001 carbonate globules: potential Martian magnetofossils. Geochim. Cosmochim. Acta 64 (23), 4049-4081.
Torchilin VP, et al. p-Nitrophenylcarbonyl-PEG-PE-liposomes: fast and simple attachment of specific ligands, including monoclonal antibodies, to distal ends of PEG chains via p-nitrophenylcarbonyl groups. Biochim Biophys Acta. 2001; 1511:397-411.
Valverde-Tercedor, C., Montalbán-López, M., Perez-Gonzalez, T., Sanchez-Quesada, M.S., Prozorov, T., Pineda-Molina, E., Fernandez-Vivas, M.A., Rodriguez-Navarro, A.B., Trubitsyn, D., Bazylinski, D.A., Jimenez-Lopez, C., 2015.Size control of in vitro synthesized magnetite crystals by the MamC protein of Magnetococcusmarinus strain MC-1. Appl. Microbiol. Biotechnol. 99, 5109-5121.

## Claims

1. A composition containing:
(i) a pure mineral phase of superparamagnetic biomimetic magnetite;
(ii) MamC; and
(iii) optionally, Mms6;
wherein at least components (i) and (ii) form superparamagnetic magnetic nanoparticles containing up to 5 wt% of MamC and with a mean particle size between 30 and 120 nm.

2. The composition according to claim 1, wherein there are no detectable levels of siderite in the composition.

3. The composition according to any of the preceding claims, **characterized in that** its goethite content does not exceed 5 wt%.

4. The composition according to any of the preceding claims, wherein the mean particle size is 30-50 nm.

5. The composition according to any of the preceding claims, wherein the magnetic biomimetic nanoparticles are functionalized with a therapeutic agent, preferably a chemotherapeutic agent, more preferably doxorubicin.

6. The composition according to any of the preceding claims, wherein the magnetic biomimetic nanoparticles are functionalized with a signaling substance, preferably a monoclonal antibody.

7. The composition according to any of the preceding claims, wherein the MamC-mediated biomimetic nanoparticles are composed of ∼95 wt% of magnetite and ∼5 wt% of MamC, with a mean particle size between 20 and 120 nm, an isoelectric point of ∼4.4, a specific surface area of ∼90 m²/g, a blocking temperature of ∼145 K, and an irreversibility temperature of ∼292 K.

8. A formulation for preparing magnetoliposomes comprising:
(i) the composition according to any of claims 1-7;
(ii) a liposome-forming agent; and
(iii) optionally, inorganic superparamagnetic magnetites (MNPs).

9. The magnetoliposome formulation according to claim 8, wherein the liposomes are functionalized with a therapeutic agent and/or a signaling substance.

10. A pharmaceutical composition comprising the composition of the present invention or the magnetoliposome formulation of the present invention and a pharmaceutically acceptable carrier and/or diluent.

11. The composition according to any of claims 1-7, the magnetoliposome formulation according to any of claims 8-9, or the pharmaceutical composition according to claim 8 for use as a medicament.

12. The composition according to any of claims 1-7, the magnetoliposome formulation according to any of claims 8-9 or the pharmaceutical composition according to claim 8 for use in the treatment of cancer.

13. The composition, the magnetoliposome formulation, or the pharmaceutical composition for use according to claim 12, wherein the cancer is selected from the group consisting of acute lymphocytic leukemia, acute myelogenous leukemia, bone cancer, breast cancer, cervical cancer, gastric cancer, head and neck tumor, Hodgkin lymphoma, Non-Hodgkin lymphoma, liver cancer, kidney cancer, multiple myeloma, neuroblastoma, ovarian cancer, non-small cell lung cancer, soft tissue sarcoma, thymomas, thyroid cancer, transitional cell bladder cancer, Wilms' tumor, and Waldenstrom macroglobulinemia.

14. A method for producing a composition of a substantially pure mineral phase of superparamagnetic biomimetic magnetite, said method comprising the following steps:
(a) preparing a carbonate solution;
(b) adding FeCl₃ to the carbonate solution;
(c) adding MamC and, optionally, Mms6 to the solution obtained in step (b);
(d) incubating the solution obtained in step (c) for at least 30 minutes;
(e) adding Fe(ClO₄)₂ to the solution obtained in step (d); and
(f) adjusting the pH of the solution obtained in step (e) to 9 using a base;
wherein the method is performed at 25°C and 1 atmosphere of pressure and all the solutions used are previously deoxygenated.

15. The method according to claim 14, wherein the carbonate solution consists of NaHCO₃ and Na₂CO₃ and, optionally, the base is NaOH.

16. The method according to claim 15, wherein the final concentration of the solution obtained in step (f) is 3.5 mM NaHCO₃, 3.5 mM Na₂CO₃, 2.78 mM Fe(ClO₄)₂, 5.56 mM FeCl₃ and a variable amount of MamC and, optionally, Mms6.

17. The method according to claim 15, wherein the concentration of the stock proteins in solution are [MamC] = 2-5 mg/mg, [Mms6] and [Mms7] >1 mg/mL.

18. Use of the composition according to any of claims 1-7, the magnetoliposome formulation according to any of claims 8-9, or the pharmaceutical composition according to claim 8 for the preparation of a contrast agent for clinical imaging techniques, preferably magnetic resonance imaging techniques.

19. Use of the composition according to any of claims 1-7, for nucleic acid isolation and purification.

20. Use of the composition according to any of claims 1-7 as a biosensor or a molecular separator.
